(19) Europäisches Patentamt
European Patent Office
Office européen des brevets

(11) **EP 3 161 145 B1**

(12) **FASCICULE DE BREVET EUROPEEN**

(45) Date de publication et mention
de la délivrance du brevet:
**20.11.2019 Bulletin 2019/47**

(21) Numéro de dépôt: **15742352.6**

(22) Date de dépôt: **29.06.2015**

(51) Int Cl.:
*G01N 21/17* (2006.01)   *C12M 1/00* (2006.01)
*C12Q 1/04* (2006.01)   *C12M 1/34* (2006.01)
*C12Q 1/18* (2006.01)

(86) Numéro de dépôt international:
**PCT/FR2015/051763**

(87) Numéro de publication internationale:
**WO 2016/001555 (07.01.2016 Gazette 2016/01)**

(54) **PROCEDE DE DETECTION D'UNE PRESENCE OU D'UNE ABSENCE DE PARTICULES BIOLOGIQUES**

VERFAHREN ZUR ERKENNUNG DES VORHANDENSEINS ODER DES FEHLENS BIOLOGISCHER PARTIKEL

METHOD FOR DETECTING A PRESENCE OR ABSENCE OF BIOLOGICAL PARTICLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2014 FR 1456117**

(43) Date de publication de la demande:
**03.05.2017 Bulletin 2017/18**

(73) Titulaire: **bioMérieux**
**69280 Marcy-l'Étoile (FR)**

(72) Inventeurs:
• **DRAZEK, Laurent**
**F-38100 Grenoble (FR)**
• **PINSTON, Frédéric**
**F-38100 Grenoble (FR)**

(56) Documents cités:
**EP-A1- 1 195 430**   **EP-A2- 0 207 739**
**EP-A2- 1 016 707**   **WO-A1-00/55357**
**DE-U1-202004 000 946**   **FR-A1- 2 958 298**
**US-A1- 2003 186 350**   **US-A1- 2010 099 137**

EP 3 161 145 B1

**Description**

L'invention porte sur un procédé de détection d'une présence ou d'une absence d'au moins une première zone d'inhibition que comprend une culture de particules biologiques sur un milieu de culture en présence d'un agent chimique, le procédé de détection comprenant une première étape d'ensemencement du milieu de culture avec les particules biologiques, une deuxième étape de réception par le milieu de culture de l'agent chimique et une troisième étape d'incubation du milieu de culture.

Le document WO 2011/125033 décrit un procédé de détection d'un amas de particules biologiques sur une surface. Les particules biologiques sont choisies parmi des microorganismes, tels que des bactéries, des levures ou des champignons, voire parmi des cellules végétales ou animales. Les particules biologiques présentent un diamètre ou une dimension principale typiquement inférieure ou égale à 100 µm. La surface est choisie parmi une interface entre un milieu de culture et un milieu environnant, tel que l'air, une surface d'un substrat fonctionnalisé ou une surface d'une membrane microporeuse.

Le procédé comporte une pluralité d'étapes dont une première étape consistant à déterminer une représentation topographique de la surface et une deuxième étape consistant à détecter sur la représentation topographique un contour délimitant une région susceptible de correspondre à un amas de particules biologiques, notamment des colonies bactériennes, ces étapes étant mises en oeuvre à l'aide d'un moyen électronique de traitement de données.

La première étape est mise en oeuvre à partir d'un procédé optique effectué sans contact et sans préparation de l'échantillon. Selon une première variante, la première étape est mise en oeuvre par microtopographie confocale chromatique. Selon une deuxième variante, la première étape est mise en oeuvre par strioscopie ou par ombroscopie. Plus particulièrement, la première étape comporte une phase d'éclairement par une source lumineuse de la surface dont une représentation tridimensionnelle doit être réalisée, une phase de détection, par un capteur de lumière, d'un signal correspondant à la lumière réfléchie ou transmise par cette surface, et une phase de détermination d'une représentation topographique tridimensionnelle de la surface à partir du signal détecté.

L'éclairement de la surface peut être dirigé du milieu ambiant vers la surface. Lorsque l'éclairement n'est pas collimaté, on peut le focaliser sur la zone à mesurer. La source de lumière peut être spatialement et/ou temporellement cohérente, ou bien être incohérente. Elle peut être polychromatique ou monochromatique. La détection peut être réalisée par un capteur d'image monodimensionnel, tel qu'une barrette, ou bidimensionnel, tel qu'une matrice, de type CMOS, CCD, photomultiplicateur, photodiode, etc... Ce capteur peut avoir une fonction spectrométrique, permettant d'analyser le spectre de la lumière détectée. Il peut aussi être couplé à un diaphragme, ou trou sténopéïque, afin de constituer un dispositif de détection confocal.

Les colonies bactériennes sont susceptibles d'être observées par un montage mettant en oeuvre une technique d'ombroscopie. Un faisceau lumineux parallèle traverse une boîte de Pétri contenant le milieu de culture, puis est focalisé vers un détecteur par une première lentille. Lorsqu'une colonie bactérienne se développe à la surface du milieu de culture, elle dévie le rayonnement lumineux, du fait de la variation d'altitude, et donc de la pente locale, formée à la surface. Le rayonnement dévié par une variation d'altitude, et/ou une variation d'indice, sur la surface observée n'est pas capté par le détecteur. Ce dispositif permet de détecter une pente locale sur une surface plane à analyser, se traduisant par l'apparition d'une zone sombre sur l'image formée par le détecteur. Ainsi, selon cette méthode, les colonies bactériennes apparaissent sous la forme de taches sombres sur un fond clair, ce dernier correspondant à la surface du milieu de culture. La représentation de la topographie de la surface du milieu est une représentation bidimensionnelle, c'est-à-dire une image, sur laquelle la variation d'intensité entre deux pixels adjacents traduit une variation d'altitude de la surface éclairée.

Dans le domaine des diagnostics de réponse de particules biologiques à un agent chimique, tel qu'un antibiotique ou analogue, il existe un besoin de disposer d'un procédé de détection d'une présence ou d'une absence de particules biologiques qui est rapide, efficace et fiable. Il existe de nombreuses méthodes d'imagerie permettant de détecter la présence ou l'absence d'une zone d'inhibition de croissance de particules biologiques en présence d'un agent chimique. Cependant, les phénomènes de diffusion de l'agent chimique à la surface et dans la gélose d'un milieu de culture, combinés à la croissance des particules biologiques, notamment des microorganismes, à la surface du milieu font qu'il est difficile de détecter une zone d'inhibition dont la valeur mesurée est stable dans le temps et peu sensible au type de milieu et d'agent chimique employé. D'une part, la concentration d'agent chimique varie dans les premières heures de diffusion jusqu'à atteindre une valeur plateau, d'autre part, les particules biologiques en présence d'un milieu de culture se développent et ce de façon dépendante à la concentration en agent chimique. Les différents types d'agent chimiques, notamment d'antibiotiques, peuvent également avoir un comportement de diffusion différent et possèdent un indice de réfraction propre. La détermination d'une frontière stable entre la zone de croissance, où les particules croissent, et la zone d'inhibition, où l'agent chimique est en concentration trop importante pour que les particules croissent, est ainsi particulièrement complexe. Pour pallier à cet inconvénient, il est ainsi indiqué dans les recommandations des organismes de régulation tels que le NCCLS (National Committee for Clinical Laboratory Standards) ou l'EUCAST (European Committee on Antimicrobial Susceptibility Testing) de procéder à une lecture unique à dix-huit heures, voire vingt heures, éventuellement à ving-quatre heures afin d'être sûr que les phénomènes de diffusion de l'agent chimique soient stabilisés dans et sur la gélose et que la valeur mesurée de la zone d'inhibition soit fiable et répétable, en comparaison aux valeurs de mesures de référence. Plus particulièrement, il est donc souhaitable de disposer d'un tel procédé dont un temps de diagnostic est amélioré, notamment inférieur à huit heures, avec une fiabilité et une répétabilité optimisées. Les documents US2003/186350 A1, EP 1 016 707 A2 et DE 20 2004 000946 U1 décrivent la détection de la présence ou l'absence de zones d'inhibition sur un milieu de culture ensemencé par des particules biologiques et sur lequel un support imprégné d'agent chimique est déposé.

Le but de la présente invention est défini par l'objet des revendications 1 à 19. Un but de la présente description est donc de proposer un procédé de détection d'une présence ou d'une absence d'une première zone d'inhibition qui est fiable, dont la répétabilité est optimisée et ce quel que soit le milieu de culture utilisé et le type d'agent chimique. D'autre part, un autre but de la description est de proposer un procédé de mesure d'une première zone d'inhibition rapide et stable dans le temps, notamment tout au long de la diffusion de l'agent chimique dans le milieu de culture. Un autre but de la description est de proposer un procédé de détection d'une présence ou d'une absence de particules biologiques qui est fiable, dont la répétabilité est optimisée et qui offre un temps de réponse relatif à une présence d'un agent chimique qui est court, notamment égal à huit heures, et plus particulièrement qui est plus rapide qu'un procédé connu de l'art antérieur qui se base sur une observation à l'œil nu des particules biologiques. Un autre but de la présente description est de proposer un dispositif de détection pour la mise en œuvre d'un tel procédé de détection.

Un procédé de la présente description est un procédé de détection d'une présence ou d'une absence d'au moins une première zone d'inhibition que comprend une culture de particules biologiques sur un milieu de culture en présence d'un agent chimique. Le procédé de détection comprend une première étape d'ensemencement du milieu de culture avec les particules biologiques, une deuxième étape de réception par le milieu de culture d'un support imprégné de l'agent chimique, une troisième étape d'incubation du milieu de culture.

Selon la présente invention, le procédé de détection comprend une quatrième étape de mesure de la première zone d'inhibition du milieu de culture qui comporte une première phase de prise d'une image de la première zone d'inhibition par ombroscopie. En particulier, la quatrième étape comporte une première phase de prise d'une image du milieu de culture par ombroscopie de sorte à obtenir une image bidimentionnelle d'intensité dudit milieu dans laquelle la première zone d'inhibition est plus sombre qu'une zone de prolifération des particules biologiques; et une analyse de l'image bidimentionnelle d'intensité de sorte à détecter la présence ou l'absence de la première zone d'inhibition

Selon une première forme de réalisation de la présente invention, la quatrième étape de mesure comprend une mesure d'une distance entre le support imprégné et un bord de la première zone d'inhibition.

Selon une deuxième forme de réalisation de la présente invention, la quatrième étape de mesure comprend une mesure d'un diamètre de la première zone d'inhibition. Dans ce mode de réalisation, cette quatrième étape peut avantageusement être suivie d'au moins une opération d'estimation d'une concentration minimale d'inhibition, pour déterminer une susceptibilité des particules biologiques envers l'agent chimique.

Le procédé de détection comprend avantageusement une cinquième étape de traitement qui comporte une deuxième phase de prise d'un cliché témoin du milieu de culture.

Le cliché témoin est par exemple le cliché d'une règle disposée sur le milieu de culture.

Le procédé de détection comprend avantageusement une troisième phase de superposition de l'image et du cliché témoin pour former une représentation normée.

La cinquième étape de traitement comprend avantageusement une quatrième phase de localisation d'un repère de la règle.

La cinquième étape de traitement comprend avantageusement une cinquième phase de traitement de la représentation normée.

La cinquième phase comprend avantageusement au moins une opération de lissage pour homogénéiser la représentation normée.

La cinquième phase comprend avantageusement au moins une opération de dilatation de la dynamique de l'intensité de pixels de la représentation normée pour former un histogramme d'un contraste de la représentation normée.

La cinquième phase comprend avantageusement au moins une opération de seuillage de la représentation normée qui comporte une détection d'un seuil et une détermination d'un contour de la représentation normée.

Le procédé de détection comprend avantageusement au moins une opération d'estimation d'une concentration minimale d'inhibition, pour déterminer une susceptibilité des particules biologiques envers l'agent chimique.

La première phase de prise de l'image et la deuxième phase de prise du cliché témoin sont par exemple des phases réalisées en continu.

La première phase de prise de l'image et la deuxième phase de prise du cliché témoin sont par exemple des phases réalisées à un laps de temps déterminé.

Des moyens de calcul de la présente invention sont des moyens de calcul configurés pour la mise en œuvre d'un tel procédé de détection, préférentiellement pour la réalisation des étapes de mesure 104 et/ou de traitement 105.

Un processeur de la présente invention comprend avantageusement de tels moyens de calcul.

Un dispositif de détection de la présente invention est principalement reconnaissable en ce que le dispositif de détection comprend un tel processeur.

Le dispositif de détection comprend avantageusement un moyen de captation, une source lumineuse apte à produire des rayons lumineux collimatés et un foyer lumineux. Un dispositif de détection selon l'invention pour la mise en œuvre d'un procédé précité, comprend :

- une source lumineuse configurée pour produire des rayons lumineux collimatés en direction d'un milieu de culture de particules biologiques;
- un foyer lumineux configuré pour éclairer directement ledit milieu de culture;
- un moyen de captation configuré pour prendre une image bidimensionnelle d'intensité dudit milieu de culture; et
- des moyens de calcul configuré pour analyser ladite image de sorte à détecter la présence ou l'absence d'au moins une première zone d'inhibition des particules biologiques sur ledit milieu de culture.

Le moyen de captation comporte avantageusement un axe de captation qui est ménagé orthogonalement par rapport à un premier plan selon lequel est étendu le milieu de culture.

La source lumineuse est avantageusement placée sur l'axe de captation, le milieu de culture étant interposé entre le moyen de captation et la source lumineuse.

Le dispositif de détection comprend avantageusement au moins un miroir semi-réfléchissant pour renvoyer les rayons lumineux émis par la source lumineuse vers le milieu de culture.

D'autres caractéristiques et avantages de la présente invention apparaîtront à la lecture de la description qui va en être faite d'exemples de réalisation, en relation avec les figures des planches annexées, dans lesquelles :

La figure 1 est une vue en coupe d'une boîte de Pétri utilisée pour la mise en oeuvre d'un procédé de détection de la présente invention.

La figure 2 est une vue schématique du procédé de détection de la présente invention.

La figure 3 est une illustration schématique de séquences du procédé de détection de la présente invention.

Les figures 4, 5 et 6 sont des illustrations schématiques de dessus de la boîte de Pétri illustrée sur la figure 1 à trois temps respectifs distincts selon un premier mode de réalisation de la présente invention.

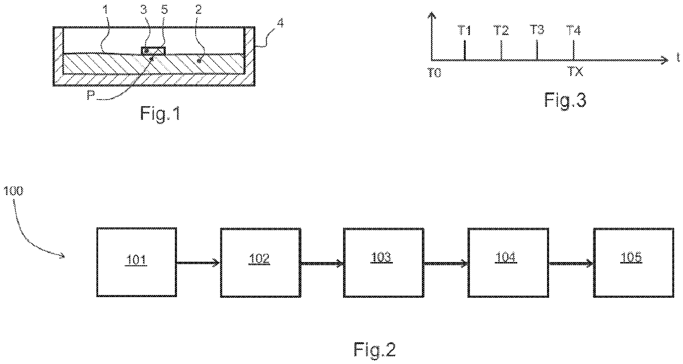

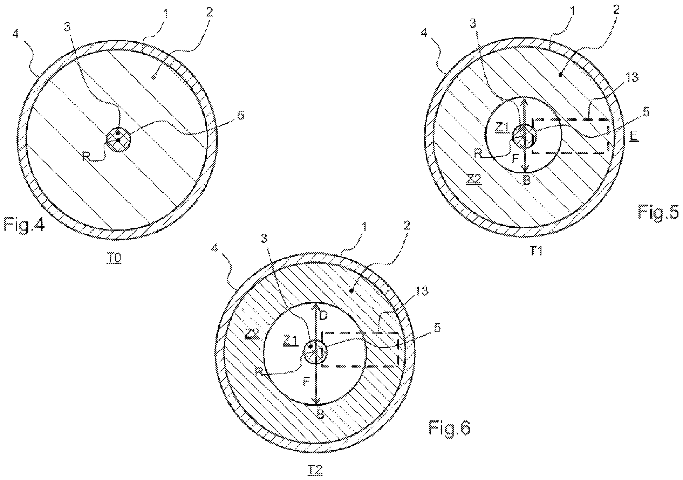

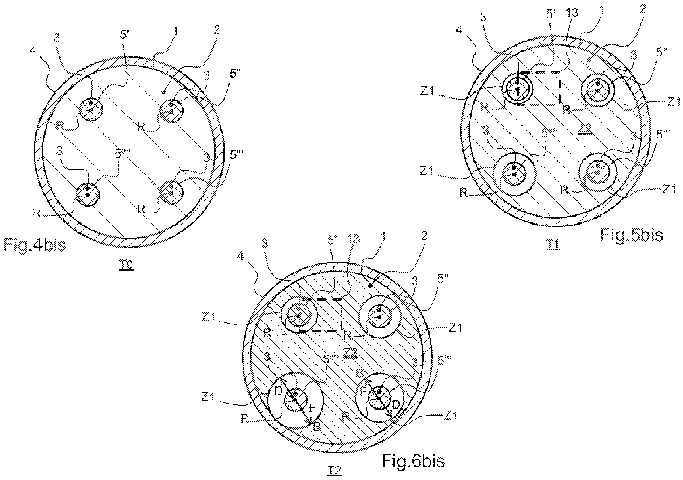

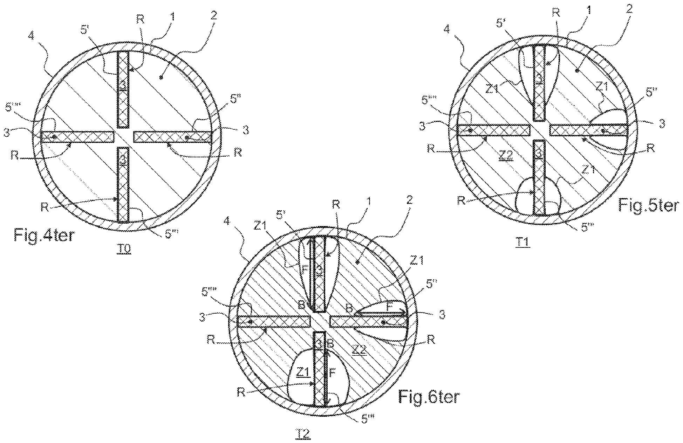

Les figures 4bis, 5bis et 6bis sont des illustrations schématiques de dessus de la boîte de Pétri illustrée sur la figure 1 à trois temps respectifs distincts selon un deuxième mode de réalisation de la présente invention.

Les figures 4ter, 5ter et 6ter sont des illustrations schématiques de dessus de la boîte de Pétri illustrée sur la figure 1 à trois temps respectifs distincts selon un troisième mode de réalisation de la présente invention.

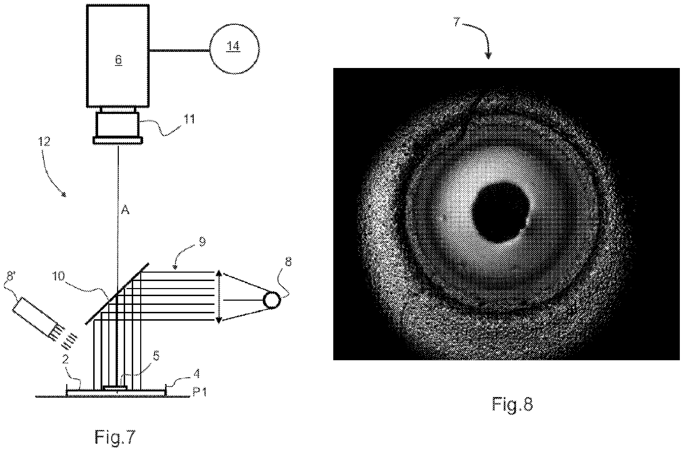

La figure 7 est une illustration schématique d'un premier mode de réalisation d'une première variante d'un dispositif de détection pour la mise en oeuvre du procédé de détection illustré sur la figure 2.

La figure 8 est une illustration d'une image obtenue à partir du dispositif de détection illustré sur la figure 7.

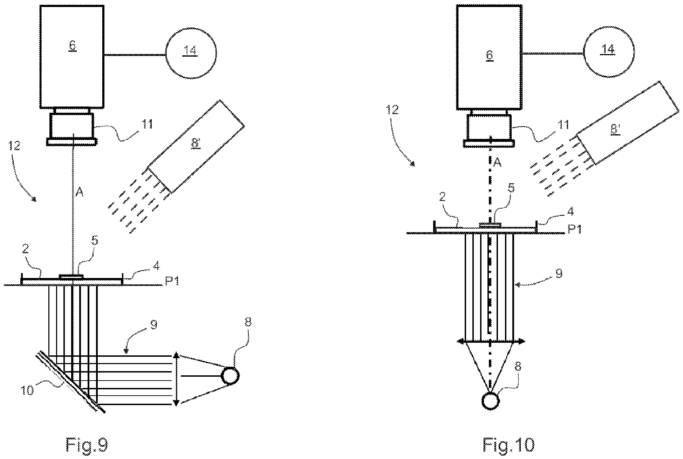

La figure 9 est une illustration schématique d'un deuxième mode de réalisation de la première variante du dispositif de détection pour la mise en oeuvre du procédé de détection illustré sur la figure 2.

La figure 10 est une illustration schématique d'une deuxième variante du dispositif de détection pour la mise en oeuvre du procédé de détection illustré sur la figure 2.

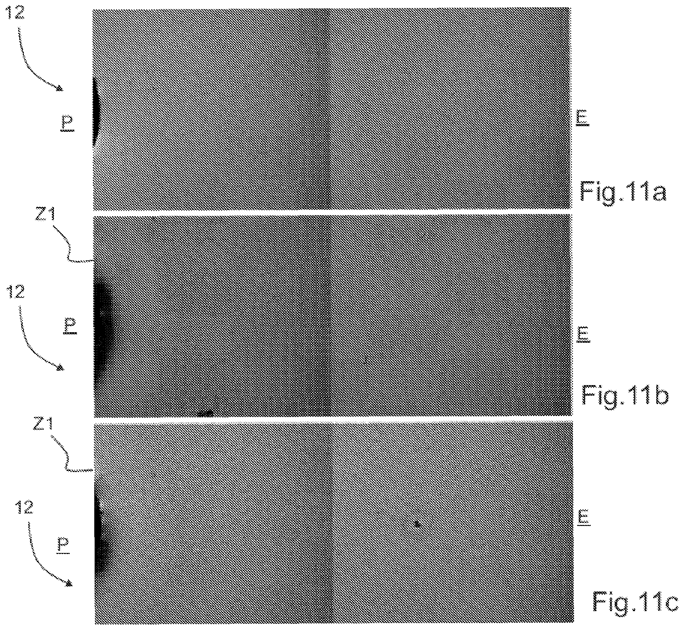

Les figures 11a, 11b et 11c sont des images prises par le dispositif de détection illustré sur la figure 10 de culture *d'Escherichia coli* (ATCC 35218) en présence respectivement d'ampicilline, d'ampicilline-sulbactam et de piperacilline-tazobactam, à un temps de mesure $T_1$ de deux heures.

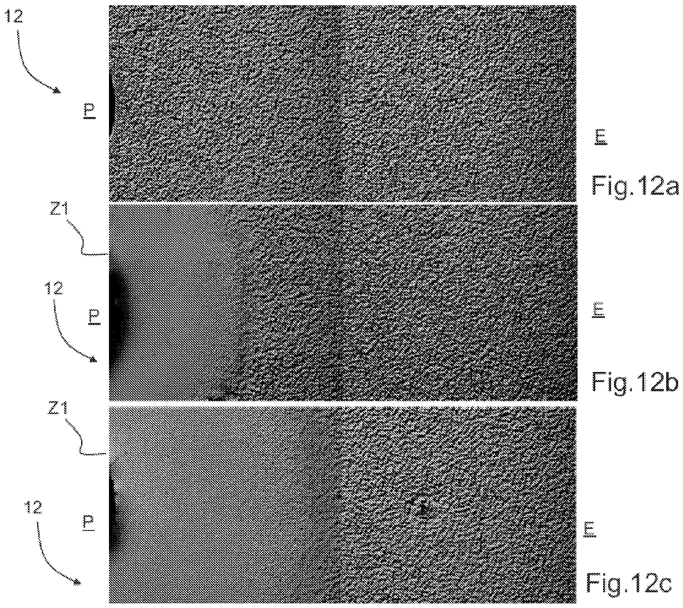

Les figures 12a, 12b et 12c sont des images prises par le dispositif de détection illustré sur la figure 10 de culture *d'Escherichia coli* (ATCC 35218) en présence respectivement d'ampicilline, d'ampicilline-sulbactam et de piperacilline-tazobactam, à un temps de mesure $T_1$ de quatre heures.

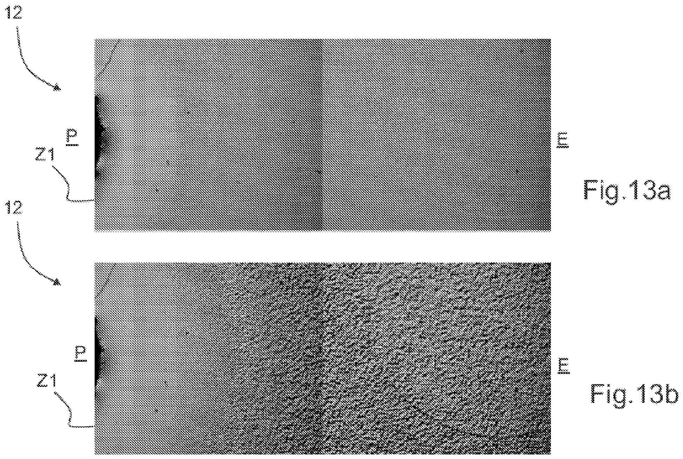

Les figures 13a et 13b sont des images prises par le dispositif de détection illustré sur la figure 10 de culture

d'*Escherichia coli* (ATCC 25922) en présence d'ampicilline, respectivement à un temps de mesure $T_1$ de deux heures et quatre heures.

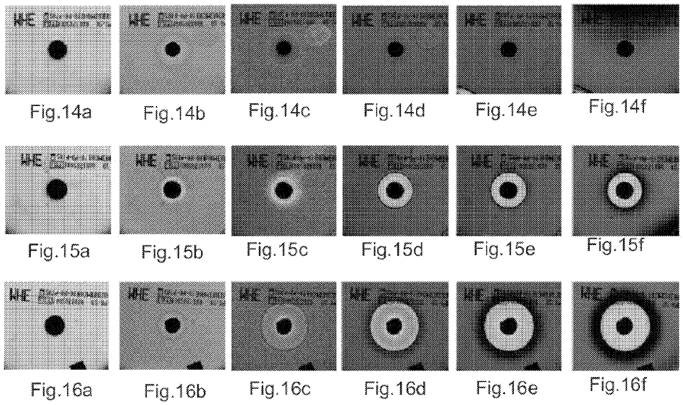

Les figures 14a, 14b, 14c, 14d, 14e et 14f sont des images prises par le dispositif de détection illustré sur la figure 9 de culture d'*Escherichia coli* (ATCC 35218) en présence d'ampicilline à un temps de mesure $T_1$ respectif de zéro heure, deux heures, quatre heures, six heures, huit heures et vingt-quatre heures.

Les figures 15a, 15b, 15c, 15d, 15e et 15f sont des images prises par le dispositif de détection illustré sur la figure 9 de culture d'*Escherichia coli* (ATCC 35218) en présence d'ampicilline-sulbactam à un temps de mesure $T_1$ respectif de zéro heure, deux heures, quatre heures, six heures, huit heures et vingt-quatre heures.

Les figures 16a, 16b, 16c, 16d, 16e et 16f sont des images prises par le dispositif de détection illustré sur la figure 9 de culture d'*Escherichia coli* (ATCC 35218) en présence de piperacilline-tazobactam à un temps de mesure $T_1$ respectif de zéro heure, deux heures, quatre heures, six heures, huit heures et vingt-quatre heures.

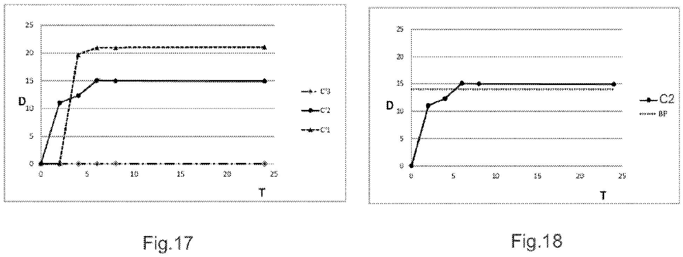

La figure 17 est une illustration schématique d'une évolution temporelle d'un diamètre de la zone d'inhibition d'*Escherichia coli* (ATCC 25922), de *Staphylococcus aureus* (ATCC 25923) et d'*Escherichia coli* (ATCC 35218) en présence d'ampicilline observées à l'aide de la mise en oeuvre du procédé de détection de la présente invention.

La figure 18 est une illustration schématique d'une évolution temporelle d'un diamètre de la zone d'inhibition d'*Escherichia coli* (ATCC 35218) en présence d'ampicilline-sulbactam observées à l'aide de la mise en oeuvre du procédé de détection de la présente invention.

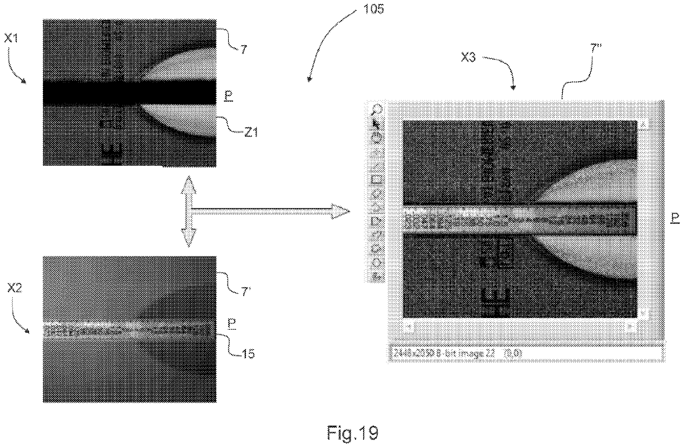

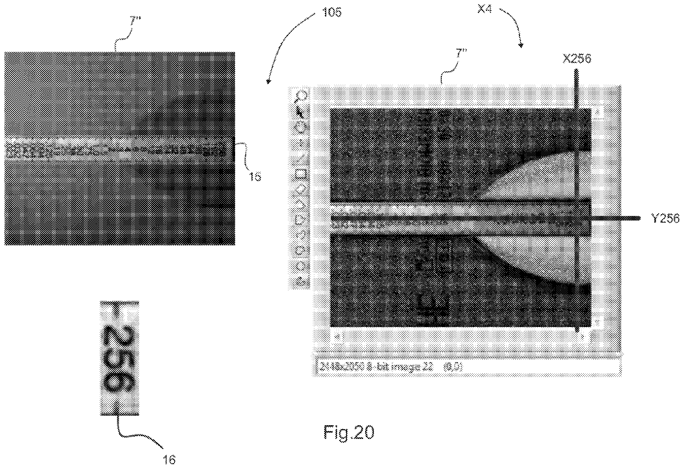

Les figures 19 et 20 sont des vues schématiques d'étapes respectives du procédé de détection illustré sur la figure 2.

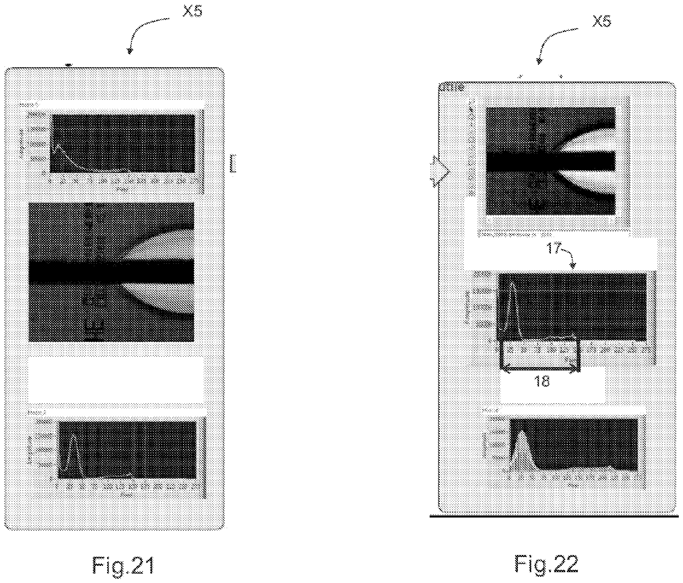

Les figures 21 à 25 sont des illustrations schématiques d'opérations successives d'une cinquième phase du procédé de détection illustré sur la figure 2.

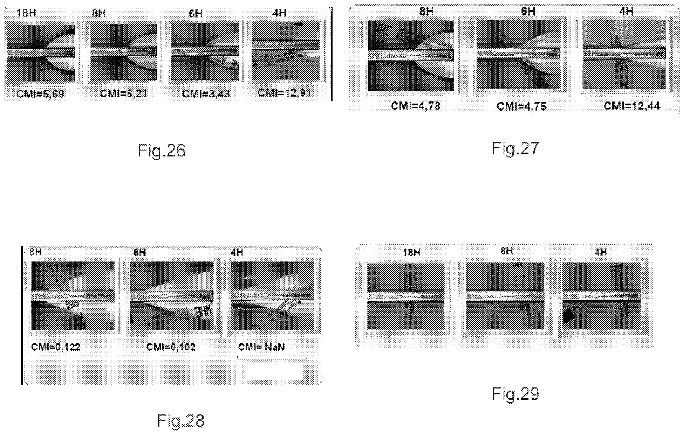

Les figures 26 à 30 sont des illustrations schématiques de représentation normées obtenues à partir de la mise en oeuvre du procédé de détection illustré sur la figure 2.

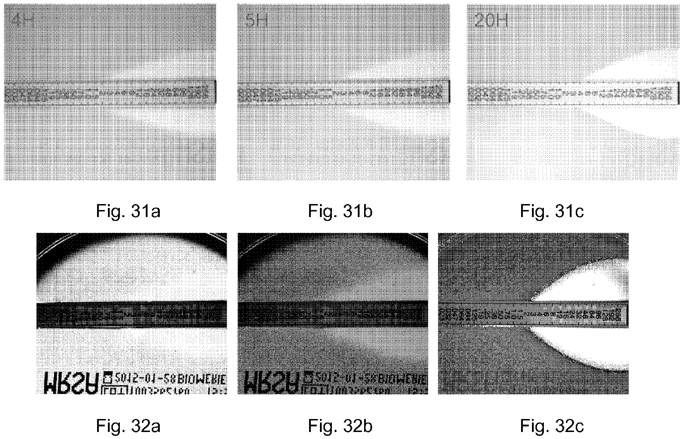

Les figures 31a, 31b, 31c, sont des images prises par le dispositif de détection illustré sur la figure 9 d'une suspension de *Staphylococcus aureus* sur un milieu de culture chromogène ChromoID™ MRSA Smart opaque (bioMérieux, Ref. 413050), en présence de céfoxitine à un temps de mesure $T_1$ respectif de quatre heures, cinq heures et vingt heures.

Les figures 32a, 32b, 32c, sont des images prises par le dispositif de détection illustré sur la figure 9 d'une suspension de *Staphylococcus aureus* sur un milieu de culture chromogène ChromoID™ MRSA transparent (bioMérieux, Ref. 43451), en présence de céfoxitine à un temps de mesure $T_1$ respectif de quatre heures, cinq heures et vingt heures.

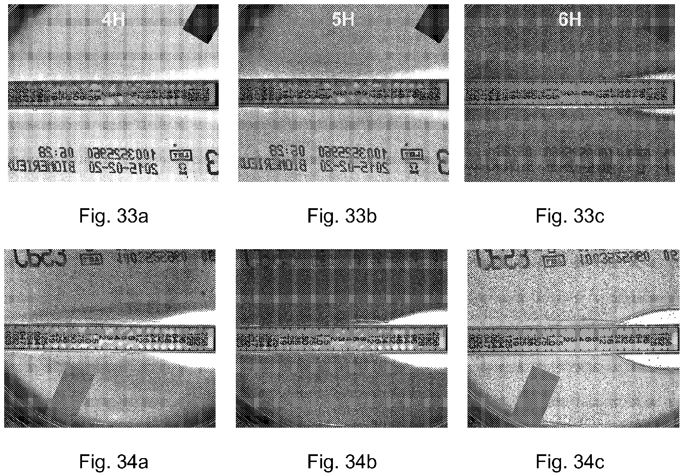

Les figures 33a, 33b, 33c, sont des images prises par le dispositif de détection illustré sur la figure 9 d'une suspension de *Staphylococcus epidermidis* sur un milieu de culture chromogène CPS® ID 3 (CPS3) (bioMérieux, Ref. 43541), en présence de Gentamicine à un temps de mesure $T_1$ respectif de quatre heures, cinq heures, six heures.

Les figures 34a, 34b, 34c, sont des images prises par le dispositif de détection illustré sur la figure 9 d'une suspension de *Staphylococcus aureus* sur un milieu de culture chromogène CPS® ID 3 (CPS3) (bioMérieux, Ref. 43541), en présence de Gentamicine à un temps de mesure $T_1$ respectif de quatre heures, cinq heures, six heures.

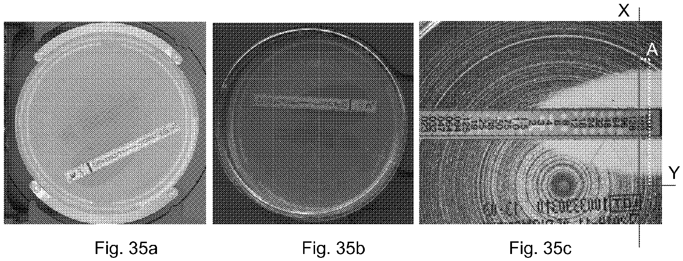

Les figures 35a, 35b, 35c, sont des images prises par différents dispositifs de détection, respectivement « SIU », « ADVENCIS » et le dispositif illustré sur la figure 9 d'une culture d'*Escherichia Coli* sur un milieu de culture Mueller Hinton E (bioMérieux MHE, Ref. 413822), en présence de Gentamicine à un temps de mesure $T_1$ de six heures.

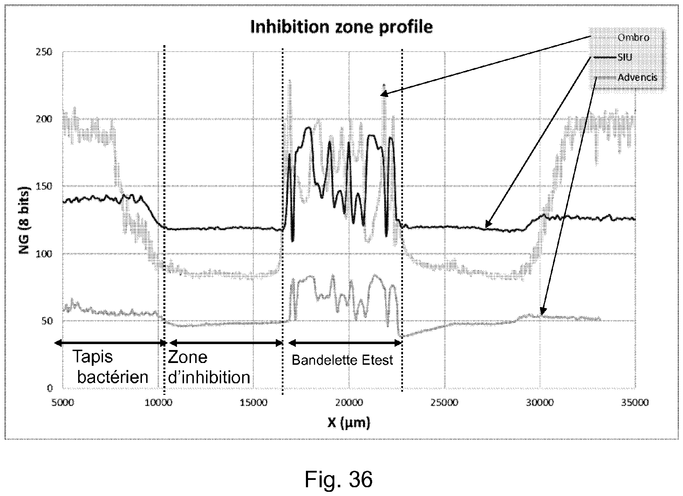

La figure 36 est une illustration schématique de trois profils de niveaux de gris observés sur les images des figures 35a, 35b et 35c, prises par trois dispositifs de détection

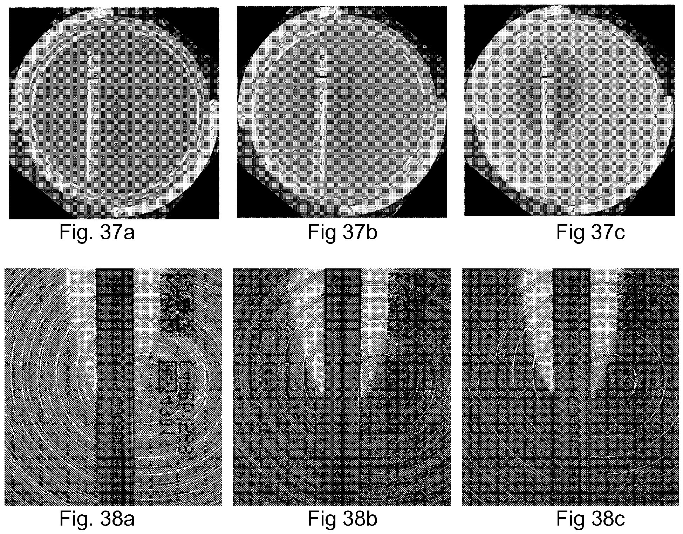

Les figures 37a, 37b, 37c, sont des images prises par un dispositif de détection, « SIU », d'une suspension d'*Escherichia Coli* (ATCC « American Type Culture Collection » 25922) sur un milieu de culture Mueller Hinton E (bioMérieux MHE, Ref. 413822), en présence de Gentamicine après incubation à un temps de mesure $T_1$ respectivement

de quatre heures, six heures et vingt-quatre heures.

Les figures 38a, 38b, 38c, sont des images prises par un dispositif de détection, par ombroscopie selon la figure 9, d'une suspension_d'*Escherichia Coli* (ATCC 25922) sur un milieu de culture Mueller Hinton E (bioMérieux MHE, Ref. 413822), en présence de Gentamicine après incubation à un temps de mesure $T_1$ respectivement de trois heures trente minutes, six heures et vingt-quatre heures.

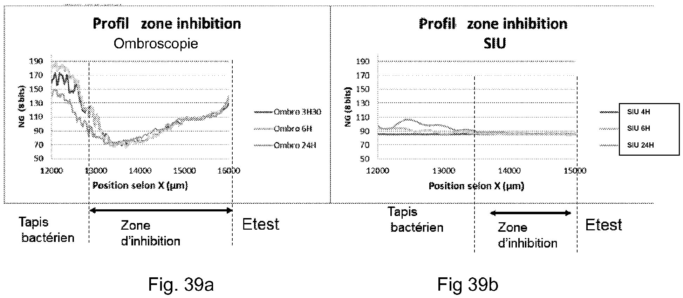

Les figures 39a et 39b sont des illustrations schématiques de trois profils de niveaux de gris observés à chaque temps de mesure à partir des deux dispositifs utilisés pour l'obtention des figures 37a, 37b et 37c et 38a, 38b, 38c.

En se reportant sur les figures 1 ou 2, dans le domaine médical et/ou pharmaceutique, il est fréquent d'avoir à utiliser un procédé de détection 100 d'une présence ou d'une absence de particules biologiques 1 sur un milieu de culture 2 au contact d'un agent chimique 3. Le milieu de culture 2 reçoit successivement les particules biologiques 1, puis l'agent chimique 3 qui est apte à inhiber un développement de certaines particules biologiques 1. Les particules biologiques 1 sont choisies indifféremment parmi des microorganismes, tels que des bactéries, des levures ou des champignons, voire parmi des cellules végétales ou animales. Le milieu de culture 2 est préférentiellement une gélose, une couche d'agar ou analogue. L'agent chimique 3 est notamment un antibiotique, un antifongique, un antimycobactérien ou un composé analogue.

Plus particulièrement, il est souhaitable de pouvoir caractériser de manière fiable et rapide une réponse des particules biologiques 1 à la présence de l'agent chimique 3, une telle réponse étant classifiée couramment selon l'une des trois affirmations suivantes : susceptible, intermédiaire ou résistant. Par « susceptible », on entend qu'une croissance, voire une survie, des particules biologiques 1 en présence de l'agent chimique 3 est impossible, à partir d'une certaine concentration d'agent chimique 3. Par « intermédiaire », on entend qu'une croissance des particules biologiques 1 en présence de l'agent chimique 3 est compromise, à partir d'une certaine concentration d'agent chimique 3. Par « résistant », on entend qu'une croissance des particules biologiques 1 en présence de l'agent chimique 3 est possible.

Un tel procédé de détection 100 trouve notamment des applications fréquentes dans le domaine des diagnostics médicaux et/ou pharmaceutiques mis en oeuvre pour la détection d'une pathologie chez un patient. Il en résulte qu'un tel procédé de détection 100 est souhaité fiable dans le sens où la nature de la réponse susvisée des particules biologiques 1 à l'agent chimique 3 est voulue certaine, sans doute et sans ambiguïté. Il en résulte aussi qu'un tel procédé de détection 100, dont des séquences successives sont illustrées sur la figure 3, est souhaité rapide avec un temps de réponse, qui s'écoule entre un temps initial $T_0$ auquel les particules biologiques 1 sont mis au contact de l'agent chimique 3 et un temps de détection $T_X$ auquel ladite réponse fiable est obtenue, qui est souhaité le plus court possible, et est notamment inférieur à huit heures. Il en résulte aussi qu'il est souhaitable qu'un tel procédé de détection 100 comporte une répétabilité idoine. De tels buts sont avantageusement atteints à partir de la mise en oeuvre du procédé de détection 100 de la présente invention.

Dans sa généralité, et en se reportant sur la figure 2, le procédé de détection 100 de la présente invention comprend une première étape d'ensemencement 101 du milieu de culture 2 avec un extrait de particules biologiques 1. L'extrait de particules biologiques 1 est par exemple un échantillon brut de particules biologiques 1 directement prélevé sur le patient. Dans ce cas-là, l'échantillon brut subit une phase de préculture permettant un isolement de souches de particules biologiques 1 en présence, dans le cas d'un échantillon comprenant une diversité de particules biologiques 1, et un accroissement des particules biologiques 1 sélectionnées et isolées. L'extrait de particules biologiques 1 est par exemple encore un échantillon préparé, notamment filtré, centrifugé et/ou purifié de manière similaire. L'échantillon de particules biologiques 1 présente une biomasse de particules biologiques 1 qui est suffisante pour être analysée valablement, tel qu'un étalon à la concentration de 0,5 Mac Farland. L'extrait de particules biologiques 1 est par exemple de l'urine, du sang, du liquide céphalorachidien ou un liquide biologique analogue.

Le milieu de culture 2 est par exemple inondé par l'extrait de particules biologiques 1, ou bien le milieu de culture 2 est ensemencé en particules biologiques 1 par écouvillonnage, ou bien encore le milieu de culture 2 reçoit les particules biologiques à partir d'un étalement à l'aide d'un râteau de ces dernières. Puis, la boîte de Pétri 4 subit des agitations transversales pour répartir de la manière la plus homogène possible l'extrait de particules biologiques 1 sur le milieu de culture 2, puis un surnageant éventuel est retiré hors de la boîte de Pétri 4. La première étape d'ensemencement 101 peut présenter des caractéristiques distinctes de celles décrites ci-dessus sans déroger aux règles de la présente invention.

Le procédé de détection 100 comprend ensuite une deuxième étape de réception 102 d'au moins une dose de l'agent chimique 3 à l'instant initial $T_0$, par exemple à partir de la mise en place d'au moins un support imprégné 5 inoculé de l'agent chimique 3 sur une zone de réception R du support imprégné 5 sur le milieu de culture 2.

Le support imprégné 5 est par exemple un disque imprégné de l'agent chimique 3, le disque étant globalement conformé en une portion cylindrique de faible épaisseur. Le disque comporte une quantité en agent chimique 3 qui est globalement homogène dans son volume.

Le support imprégné 5 est par exemple encore une bande longiligne, de conformation globalement rectangulaire. La bande comporte par exemple une concentration en agent chimique 3 qui suit un gradient croissant de concentration d'un petit bord à un petit bord opposé de la bande.

Le support imprégné 5 est par exemple encore réduit à une gouttelette d'eau contenant l'agent chimique 3.

Selon une variante de réalisation illustrée sur les figures 4, 5 et 6, le milieu de culture 2 reçoit un unique support imprégné 5. La zone de réception R est préférentiellement formée d'un point central de la boîte de Pétri 4.

Selon une autre variante de réalisation illustrée sur les figures 4bis, 5bis et 6bis, le milieu de culture 2 reçoit une pluralité de supports imprégnés 5',5",5"',5"", chaque support imprégné 5',5",5"',5"" comportant par exemple une concentration en agent chimique 3 propre et distincte des autres supports imprégnés 5',5",5"',5"". Par exemple, un premier support imprégné 5' comporte une première concentration en agent chimique 3 qui est inférieure à une deuxième concentration en agent chimique 3 que comporte un deuxième support imprégné 5", la deuxième concentration étant inférieure à une troisième concentration en agent chimique 3 que comporte un troisième support imprégné 5"', la troisième concentration étant inférieure à une quatrième concentration en agent chimique 3 que comporte un quatrième support imprégné 5"". Les zones de réception R sont en pluralité et sont par exemple formées de points équidistants du milieu de culture 2. Selon une autre variante de réalisation, chaque support imprégné 5',5",5"',5"" comporte par un agent chimique 3 distinct de celui des autres supports imprégnés 5',5",5"',5"".

Selon encore une autre variante de réalisation illustrée sur les figures 4ter, 5ter et 6ter, le milieu de culture 2 reçoit une pluralité de supports imprégnés 5',5",5"',5"", qui sont chacun conformés en bande et qui sont imprégnés d'un agent chimique respectif 3. Les zones de réception R sont en pluralité et sont par exemple conformées selon des rayons respectifs de la boîte de Pétri 4.

Ces variantes visent, selon les cas, à optimiser une efficacité et une rapidité d'analyse de la réponse des particules biologiques 1 à divers agents chimiques 3 et à différentes concentrations de ce dernier, avec une cadence d'analyse optimisée. Selon ces diverses variantes, il résulte de la deuxième étape de réception 102 une délivrance instantanée de l'agent chimique 3 à l'intérieur du milieu de culture 2 qui se propage à l'intérieur du milieu de culture 2 depuis le support imprégné 5,5',5",5"',5"".

Le procédé de détection 100 comprend ensuite une troisième étape d'incubation 103 au cours de laquelle les particules biologiques 1 interagissent avec l'agent chimique 3 au cours d'un laps de temps défini correspondant à un temps d'incubation. Préférentiellement, une telle étape d'incubation 103 est effectuée à température constante, par exemple de 37°C, dans un environnement contrôlé. Le temps d'incubation est préférentiellement de l'ordre de quelques heures, et notamment de deux heures, ou bien quatre heures, ou bien six heures, ou bien huit heures, ou bien dix-huit heures, ou bien vingt heures, voire vingt-quatre heures. Typiquement, le temps d'incubation est équivalent aux temps d'observation $T_1$, $T_2$ décrits ci-dessous.

En se reportant respectivement sur les figures 5, 5bis, 5ter d'une part et sur les figures 6, 6bis, 6ter d'autre part, il résulte de ces dispositions, lors d'une observation à un premier temps d'observation $T_1$, puis à un deuxième temps d'observation $T_2$, une formation d'au moins une première zone d'inhibition $Z_1$ des particules biologiques 1 à l'intérieur de laquelle les particules biologiques 1 sont inhibées, dans le cas favorable où ces dernières sont sensibles à l'agent chimique 3. Dans ce cas, soit les particules biologiques 1 sont détruites, soit leur croissance est arrêtée. Dans le cas inverse, le milieu de culture 2 reste homogène avec une concentration en particules biologiques 1 globalement homogène à l'intérieur de l'ensemble du milieu de culture 2. Dans ce cas, on observe une absence de zone d'inhibition $Z_1$, autrement dit, on observe une pousse de particules biologiques 1 en présence de l'agent chimique 3.

Sur les figures 5,5bis et sur les figures 6,6bis, les zones d'inhibition $Z_1$ sont globalement circulaires et centrées sur la zone de réception R. Chaque zone d'inhibition $Z_1$ des particules biologiques 1 tend à s'accroître radialement depuis la zone de réception R, entre le premier temps d'observation $T_1$ illustré sur les figures 5, 5bis, et le deuxième temps d'observation $T_2$ illustré sur les figures 6, 6bis.

Sur la figure 5ter et sur la figure 6ter, les zones d'inhibition $Z_1$ sont globalement ellipsoïdales et réparties symétriquement de part et d'autre de la zone de réception R. Chaque zone d'inhibition $Z_1$ des particules biologiques 1 tend à s'accroître de manière centrifuge depuis la zone de réception R, entre le premier temps d'observation $T_1$ illustré sur la figure 5ter et le deuxième temps d'observation $T_2$ illustré sur la figure 6ter.

Le procédé de détection 100 comprend ensuite une quatrième étape de mesure 104 de la première zone d'inhibition $Z_1$ par ombroscopie. La quatrième étape de mesure 104 comprend une phase de mesure d'une distance F qui s'étend entre la zone de réception R et un bord extrême B de la zone d'inhibition $Z_1$.

Sur les figures 6 et 6bis, la distance F est équivalente au diamètre D de la zone d'inhibition $Z_1$.

Sur la figure 6ter, la distance F est équivalente à une longueur de la zone d'inhibition Z1 prise le long du support imprégné 5.

En se reportant sur les figures 7, 9 et 10, la quatrième étape de mesure 104 met en oeuvre un moyen de captation 6 de l'image 7 et une source lumineuse 8. Dans sa généralité, la source lumineuse 8 éclaire la boîte de Pétri 4 dont l'image 7 est prise soit à intervalle temporel régulier par le moyen de captation 6, soit après un laps de temps déterminé propre à chaque boîte de Pétri 4, soit en continu sur une même boîte de Pétri 4. Le moyen de captation 6

comporte un axe de captation A qui est préférentiellement ménagé orthogonalement par rapport à un premier plan P1 selon lequel est étendu le milieu de culture 2. Le moyen de captation 6 surplombe avantageusement la boîte de Pétri 4 de manière à prendre une vue de dessus du milieu de culture 2. Le moyen de captation 6 est par exemple une caméra CCD, notamment de type Basler piA2400 - 17 gm, qui est équipée d'un objectif télécentrique 11. La source lumineuse 8 est préférentiellement un illuminateur collimaté apte à produire des rayons lumineux 9 parallèles entre eux qui atteignent orthogonalement le milieu de culture 2. La source lumineuse 8 comprend une pluralité de diodes comportant une gamme d'émission indifféremment dans le rouge, le vert, le bleu et le blanc.

Selon un premier mode de réalisation d'une première variante illustrée sur la figure 7, la source lumineuse 8 est disposée latéralement au-dessus de la boîte de Pétri 4. Selon un deuxième mode de réalisation de la première variante illustrée sur la figure 9, la source lumineuse 8 est disposée latéralement au-dessous de la boîte de Pétri 4. La source lumineuse 8 est associée à un foyer lumineux 8' qui permet un éclairage direct de la boîte de Pétri 4. Selon la première variante de réalisation, la source lumineuse 8 est associée à un miroir semi-réfléchissant 10 incliné à 45° pour renvoyer des rayons lumineux 9 émis par la source lumineuse 8 vers la boîte de Pétri 4. La source lumineuse 8 est par exemple du type Opto Engineering - LTCL 048 - W. Le foyer lumineux 8' est par exemple du type Opto Engineering - LTCL 24. Dans les deux cas, l'objectif télécentrique 11 est notamment de type Opto Engineering - TC23 048, comportant un champ focal de 46x38,5 mm et une distance de travail de 134,6 mm.

Selon une deuxième variante de réalisation illustré sur la figure 10, la source lumineuse 8 est centrée en-dessous de la boîte de Pétri 4 pour adresser directement les rayons lumineux 9 émis par la source lumineuse 8 vers la boîte de Pétri 4. Plus particulièrement, la source lumineuse 8 est disposée axialement par rapport à l'axe de captation A de manière à délivrer un retro-éclairage à la boîte de Pétri 4. Dans ce cas, la source lumineuse 8 est par exemple du type Opto Engineering - LTCL 024 - G dont la longueur d'onde d'émission est de 520 nm et l'objectif télécentrique 11 est par exemple du type Edmund Optics - 63733, comportant un champ focal de 8,44 x 7,07 mm et une distance de travail de 65 mm.

Le moyen de captation 6, la source lumineuse 8 et éventuellement le miroir semi-réfléchissant 10 et le foyer lumineux 8' forment conjointement un dispositif de détection 12 apte à mettre en oeuvre le procédé de détection 100 de la présente invention.

En se reportant sur les figures 5, 6 ,5bis, 6bis, 5ter, 6ter et 8, lorsque les rayons lumineux 9 traversent les premières zones d'inhibition $Z_1$ du milieu de culture 2 exemptes de particules biologiques 1, les rayons lumineux 9 traversent le milieu de culture 2 sans être déviés, de telle sorte que l'image 7 prise par le moyen de captation 6 révèle une première portion de l'image 7 qui est claire, notamment blanche, et qui est de conformation identique aux premières zones d'inhibition $Z_1$. Lorsque les rayons lumineux 9 traversent une deuxième zone de prolifération $Z_2$ comportant des particules biologiques 1, ces dernières dévient les rayons lumineux 9, de telle sorte que l'image 7 prise par le moyen de captation 6 révèle une deuxième portion de l'image 2 qui est sombre, notamment noire, et qui est de conformation identique aux deuxièmes zones de prolifération $Z_2$. Il en ressort qu'à un temps d'observation donné, un contraste entre une première zone d'inhibition $Z_1$ et une deuxième zone de prolifération $Z_2$ est supérieur au même contraste observé avec une méthode classique de l'art antérieur. On notera à ce stade de la description que l'image 7 illustrée sur la figure 8 est obtenue à l'aide du dispositif de détection 12 illustré sur la figure 7, les particules biologiques étant *Staphylococcus aureus* (ATCC 25923), l'agent chimique 3 étant la ciprofloxacine et le temps de détection $T_X$ étant de 18h.

En se reportant sur les figures 11a, 11b et 11c qui sont des images 7 de culture d'*Escherichia coli* (ATCC 35218) en présence respectivement d'ampicilline, d'ampicilline-sulbactam et de piperacilline-tazobactam, à un temps de mesure $T_1$ de deux heures, et sur les figures 12a, 12b et 12c qui sont des images 7 de culture d'*Escherichia coli* (ATCC 35218) en présence respectivement d'ampicilline, d'ampicilline-sulbactam et de piperacilline-tazobactam, à un temps de mesure $T_1$ de quatre heures ainsi que sur les figures 13a et 13b qui sont des images 7 de culture d'*Escherichia coli* (ATCC 25922) en présence d'ampicilline, respectivement à un temps de mesure $T_1$ de deux heures et quatre heures, les dites images 7 sont des images partielles de la boîte de Pétri 4 constituées d'une bandelette 13 qui est radiale de cette dernière, la bandelette 13 s'étendant entre la zone de réception R et une extrémité périphérique E de la boîte de Pétri 4. Dans un tel cas, le procédé de détection 100 est un procédé de détection par micro-ombroscopie qui présente l'avantage d'offrir une meilleure résolution et une détection plus rapide de la première zone d'inhibition $Z_1$ Ainsi, sur les figures 12b, 12c et 13b, la première zone d'inhibition $Z_1$ est identifiable à un temps de détection $T_X$ de quatre heures, voire sur les figures 11b, 11c et 13a à un temps de détection $T_X$ de deux heures. Il en résulte une réponse fiable et certaine relative à la susceptibilité d'*Escherichia coli* (ATCC 35218) au mélange ampicilline-sulbactam et au mélange de piperacilline-tazobactam, à la résistance d'*Escherichia coli* (ATCC 35218) à l'ampicilline avec un temps de détection $T_X$ de quatre heures. Il en résulte une réponse fiable et certaine relative à la susceptibilité d'*Escherichia coli* (ATCC 25922) à l'ampicilline avec un temps de détection $T_X$ de deux heures, une telle réponse étant comparable à une méthode de référence de l'art antérieur, telle qu'une méthode d'observation à l'œil nu, mais dans un temps de détection beaucoup plus court.

En se reportant sur les figures 14a, 14b, 14c, 14d, 14e et 14f qui sont des images 7 de culture d'*Escherichia coli* (ATCC 35218) en présence d'ampicilline à un temps de mesure $T_1$ respectif de zéro heure, deux heures, quatre

# EP 3 161 145 B1

heures, six heures, huit heures et vingt-quatre heures, il est remarquable qu'une réponse fiable et certaine de la résistance d'*Escherichia coli* (ATCC 35218) à l'ampicilline est identifiable dès quatre heures. En effet, aucune zone d'inhibition n'est détectée dès quatre heures et ce de façon fiable et répétable dans le temps.

En se reportant sur les figures 15a, 15b, 15c, 15d, 15e et 15f qui sont des images 7 de culture d'Escherichia coli (ATCC 35218) en présence d'ampicilline-sulbactam à un temps de mesure T1 respectif de zéro heure, deux heures, quatre heures, six heures, huit heures et vingt-quatre heures, il est remarquable qu'une réponse fiable et certaine de la susceptibilité d'Escherichia coli (ATCC 35218) au mélange ampicilline-sulbactam est identifiable dès quatre heures. En effet, la zone d'inhibition détectée dès quatre heures présente un diamètre stable et mesurable de façon répétable dans le temps.

En se reportant sur les figures 16a, 16b, 16c, 16d, 16e et 16f qui sont des images 7 de culture d'*Escherichia coli* (ATCC 35218) en présence de piperacilline-tazobactam à un temps de mesure $T_1$ respectif de zéro heure, deux heures, quatre heures, six heures, huit heures et vingt-quatre heures, il est remarquable qu'une réponse fiable et certaine de la susceptibilité d'*Escherichia coli* (ATCC 35218) au mélange ampicilline-sulbactam est identifiable dès quatre heures. En effet, la zone d'inhibition détecté dès quatre heures présente un diamètre stable et mesurable de façon répétable dans le temps. Les exemples des figures 14 à 16 montrent l'intérêt de l'utilisation de l'ombroscopie pour la détection de zones d'inhibition, ce qui démontre l'intérêt de l'utilisation de l'ombroscopie dans ce procédé, plus particulièrement de l'utilisation d'un objectif télécentrique, préférentiellement en combinaison avec un illuminateur collimaté.

En se reportant à nouveau sur la figure 2, les réponses susvisées des différentes particules biologiques 1 aux agents chimiques 3 sont obtenues à partir du procédé de détection 100 de la présente invention qui comprend avantageusement une cinquième étape de traitement 105 des images 7 obtenues par le dispositif de détection 12 de la présente invention. La cinquième étape de traitement 105 est réalisée à partir de la mise en oeuvre de moyens de calcul 14 configurés pour la réalisation de cette étape et comprenant par exemple des moyens d'analyse et de traitement d'images, les moyens de calcul 14 étant constitutifs d'un processeur que comprend le dispositif de détection 12.

Selon une première approche de la cinquième étape de traitement 105, cette dernière comprend par exemple un filtrage gaussien des images 7, une acquisition de profils respectifs des images et une dérivation de tels profils.

Selon une deuxième approche de la cinquième étape de traitement 105, cette dernière comprend par exemple un repérage de la zone de réception R, une mise de l'image en grande dynamique, un filtrage médian de cette dernière, une acquisition de profils par rotation de l'image autour de la zone de réception R, une dérivation de tels profils, un calcul d'une moyenne de profils pour déterminer la présence ou non d'une première zone d'inhibition $Z_1$ à l'intérieur de chacune des images 7.

Selon l'une ou l'autre des approches susvisées, le procédé de détection 100 permet de déterminer des cinétiques de réactions d'inhibition représentant le diamètre D de la première zone d'inhibition $Z_1$ en fonction du temps t, ces cinétiques étant illustrées sur les figures 17 et 18. Sur la figure 17, les courbes $C'_1$, $C'_2$, $C'_3$ illustrent respectivement les cinétiques des réactions d'inhibition des figures 14a à 14f, des figures 15a à 15f et des figures 16a à 16f. Sur la figure 18, la courbe $C_2$ illustre une cinétique de la réaction d'inhibition d'*Escherichia coli* (ATCC 35218) en présence de l'antibiotique ampicilline-sulbactam, par rapport à un seuil de référence BP pour les entérobactéries vis-à-vis de cet antibiotique. Après six heures, le diamètre D mesuré est supérieur au seuil de référence BP spécifié. Il en résulte que cette souche est considérée comme étant sensible à cet antibiotique.

Une troisième approche de la cinquième étape de traitement 105 est illustrée sur les figures 19 à 30.

Sur la figure 19, la cinquième étape de traitement 105 comprend une deuxième phase $X_2$ de prise d'un cliché témoin 7' d'une règle 15 disposée sur le milieu de culture 2 contenue à l'intérieur de la boîte de Pétri 4, la deuxième phase $X_2$ étant également réalisée par le dispositif de détection 12. Puis, la cinquième étape de traitement 105 comprend une troisième phase $X_3$ de superposition de l'image 7 et du cliché témoin 7' et plus particulièrement de leur zone de réception R respective pour former une représentation normée 7" du milieu de culture 2.

Sur la figure 20, la cinquième étape de traitement 105 comprend une quatrième phase $X_4$ de localisation d'un repère 16 de la règle 15. Sur l'exemple illustré, le repère 16 est l'indication « 256 » de la règle 15, le repère 16 étant susceptible d'être constitué d'une indication autre et relativement quelconque. Le repère 16 comporte des coordonnées cartésiennes $X_{256}$ et $Y_{256}$.

Sur les figures 21 à 25, la cinquième étape de traitement 105 comprend une cinquième phase $X_5$ de traitement de la représentation normée 7".

Sur la figure 21, la cinquième phase $X_5$ comprend au moins une opération de lissage pour homogénéiser la représentation normée 7". Une telle opération de lissage est par exemple réalisée à partir d'un filtrage gaussien de la représentation normée 7". De préférence, l'opération de lissage est réalisée à plusieurs reprises, sept fois notamment.

Sur la figure 22, la cinquième phase $X_5$ comprend au moins une opération de dilatation de la dynamique de l'intensité de pixels de la représentation normée 7" pour former un histogramme 17 d'un contraste de la représentation normée 7", le contraste étant à considérer entre pixels sombres et pixels clairs de la représentation normée 7". Il en

9

résulte une détermination d'une dynamique utile 18 de la représentation normée 7".

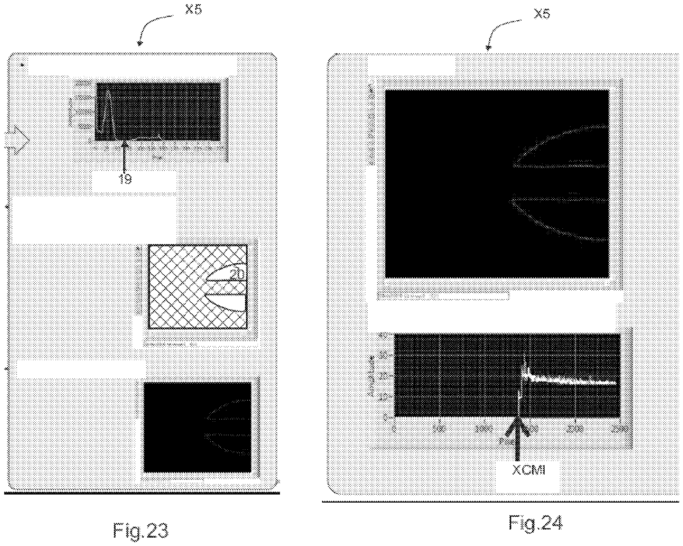

Sur la figure 23, la cinquième phase $X_5$ comprend au moins une opération de seuillage de la représentation normée 7" qui comprend par exemple une détection d'un seuil 19 et la détermination d'un contour 20 à partir d'une binarisation de la représentation normée 7".

Sur la figure 24, la cinquième phase $X_5$ comprend au moins une opération d'estimation d'une concentration minimale d'inhibition, couramment dénommée selon l'acronyme CMI, à partir de la détermination d'une abscisse de la concentration minimale d'inhibition repérée $X_{CMI}$.

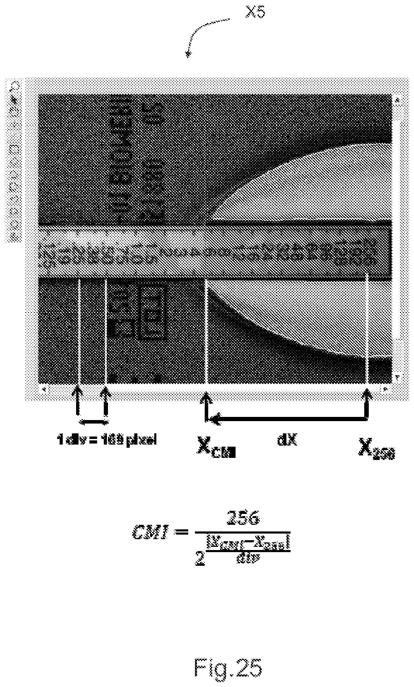

Sur la figure 25, la cinquième phase $X_5$ comprend au moins une opération de calcul de la concentration minimale d'inhibition CMI à partir de la relation [1] suivante :

$$CMI = \frac{256}{2^{\frac{|X_{CMi}-X_{256}|}{div}}} \qquad [1]$$

Avec une constante « div » qui correspond à un nombre de pixels entre deux graduations de la règle 15 séparées d'un facteur 2. La constante « div » est par exemple égal à cent soixante-neuf pixels.

L'ensemble de ces dispositions est tel que la concentration minimale d'inhibition CMI, qui est la concentration d'agent chimique 3 à partir de laquelle la première zone d'inhibition $Z_1$ apparait, est déterminée avec précision, avec fiabilité, avec répétabilité et avec un temps de réponse fiable qui est court, notamment inférieur ou égal à huit heures.

Sur les figures 26 et 27, sont représentées des représentations normées 7" de culture d'*Escherichia coli* (ATCC 25922) en présence d'ampicilline pour lesquelles il est remarquable qu'à partir de six heures la concentration minimale CMI calculée permet de caractériser la réponse d'*Escherichia coli* (ATCC 25922) comme susceptible à l'ampicilline.

Sur la figure 28, est représentée la représentation normée 7" de culture de *Staphylococcus aureus* (ATCC 25923) en présence d'ampicilline pour laquelle il est remarquable qu'à partir de six heures la Concentration minimale CMI calculée permet de caractériser la réponse de *Staphylococcus aureus* (ATCC 25923) comme susceptible à l'ampicilline.

Sur la figure 29, est représentée la représentation normée 7" de culture d'*Escherichia coli* (ATCC 35218) en présence d'ampicilline pour laquelle il est remarquable que *Escherichia coli* (ATCC 35218) est résistante à l'ampicilline en raison d'absence de première zone d'inhibition $Z_1$.

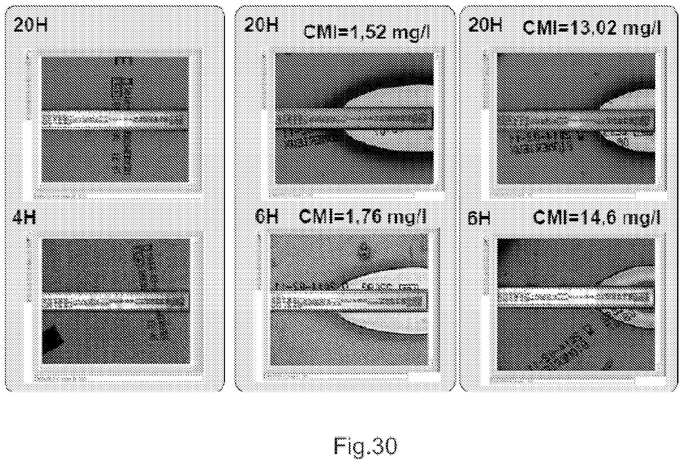

Sur la figure 30, sont représentées successivement en colonne depuis la gauche vers la droite les représentations normées 7" de culture d'*Escherichia coli* (ATCC 35218) en présence respectivement d'ampicilline, d'un mélange de piperacilline et de tazobactam et d'un mélange d'ampicilline et de sulbactam pour lesquelles il est remarquable que *Escherichia coli* (ATCC 35218) est à la fois résistant à l'ampicilline, susceptible au mélange de piperacilline et de tazobactam et intermédiaire au mélange d'ampicilline et de sulbactam, selon l'organisme EUCAST pour EUropean Committee on Antimicrobial Susceptibility Testing.

L'ensemble de ces dispositions est tel qu'à partir de la mise en oeuvre du procédé de détection 100 de la présente invention, une première zone d'inhibition $Z_1$ est fiablement détectable quatre heures après la mise en présence des particules biologiques 1 et de l'agent chimique 3 en cas de susceptibilité des premières avec le dernier.

Les figures 31a, 31b, 31c, sont des images prises par le dispositif de détection illustré sur la figure 9 d'une suspension *Staphylococcus aureus* sur un milieu de culture chromogène ChromoID™ MRSA Smart opaque (bioMérieux, Ref. 413050). L'ensemencement est réalisé par inondation avec une solution calibrée à 0.5 Mc Farland, une bandelette Etest® (bioMérieux) contenant un gradient de concentration de céfoxitine est ensuite disposée à la surface de milieu. Le milieu est incubé à 37°C et des images sont capturées à un temps de mesure $T_1$ respectif de quatre heures, 31a, cinq heures, 31b et enfin vingt heures, 31c.

Les figures 32a, 32b, 32c, sont des images prises par le dispositif de détection illustré sur la figure 9 d'une suspension de *Staphylococcus aureus* sur un milieu de culture chromogène ChromoID™ MRSA transparent (bioMérieux, Ref. 43451). L'ensemencement est réalisé par inondation avec une solution calibrée à 0.5 Mc Farland, une bandelette Etest® (bioMérieux) contenant un gradient de concentration de céfoxitine est ensuite disposée à la surface de milieu. Le milieu est incubé à 37°C et des images sont capturées à un temps de mesure $T_1$ respectif de quatre heures, 32a, cinq heures, 32b, vingt heures, 32c.

Les figures 33a, 33b, 33c, sont des images prises par le dispositif de détection illustré sur la figure 9 d'une suspension de *staphylococcus epidermidis* sur un milieu de culture chromogène CPS® ID 3 (CPS3) (bioMérieux, Ref. 43541). L'ensemencement est réalisé par inondation avec une solution calibrée à 0.5 Mc Farland, une bandelette Etest® (bioMérieux) contenant un gradient de concentration de gentamicine est ensuite disposée à la surface de milieu. Le milieu est incubé à 37°C et des images sont capturées à un temps de mesure $T_1$ respectif de quatre heures, 33a, cinq heures, 33b, et six heures, 33c.

Les figures 34a, 34b, 34c, sont des images prises par le dispositif de détection illustré sur la figure 9 d'une suspension de *staphylococcus aureus* sur un milieu de culture chromogène CPS® ID 3 (CPS3) (bioMérieux, Ref. 43541). L'ensemencement est réalisé par inondation avec une solution calibrée à 0.5 Mc Farland, une bandelette Etest® (bio-Mérieux) contenant un gradient de concentration de gentamicine est ensuite disposée à la surface de milieu. Le milieu est incubé à 35°C et des images sont capturées à un temps de mesure $T_1$ respectif de quatre heures, 34a, cinq heures, 34b, et six heures, 34c.

Les valeurs de concentration minimales d'inhibition obtenues à partir des figures 31 à 34 dès 4 heures sont conformes aux recommandations EUCAST pour les microorganismes correspondant et ce pour une lecture classique après vingt heures d'incubation. Il est également remarquable que les valeurs de concentration minimale d'inhibition lisibles dès quatre heures sur les figures 31a, 32a, 33a et 34a sont également similaires aux valeurs lisibles dès cinq heures sur les figures 31b, 32b, 33b et 34b. De même les zones d'inhibition observables sont suffisamment stables et répétables pour que les valeurs à six heures, 33c, 34c et vingt heures 31c, 32c soient également similaires. De ce fait l'intérêt de l'utilisation de l'ombroscopie, plus particulièrement à l'aide d'un objectif télécentrique préférentiellement couplé à un illuminateur collimaté, est démontrée pour une lecture à moins de huit heures, voire à moins de six heures, préférentiellement à quatre heures d'une valeur de concentration minimale inhibitrice fiable et répétable et ce pour tous types de milieux de cultures, notamment de milieux chromogéniques. Il est également démontré l'intérêt de cette technique pour la prise d'image sur des milieux opaques, figures 31a, 31b, 31c, ces milieux étant connus pour être difficile à analyser. En effet l'opacité du milieu augmente la diffusion de tout éclairage à travers la gélose et diminue ainsi le contraste de l'image capturée. Il est ainsi observé que toutes les images capturées sur les figures 31 à 34 présentent un contraste suffisamment élevé entre les zones de croissance bactérienne et les zones d'inhibition pour pouvoir aisément analyser l'image par la mise en oeuvre de moyens de calcul comprenant par exemple des moyens d'analyse et de traitement d'images, les moyens de calcul étant constitutifs d'un processeur que comprend le dispositif de détection 12.

Enfin, ces tests réalisés sur deux types d'antibiotiques différents, à savoir un bactéricide (céfoxitine) et un bactériostatique (gentamicine) permettent également de montrer la capacité du procédé selon l'invention à obtenir une valeur d'inhibition de façon rapide, dès quatre heures et en moins de huit heures, et ce de façon répétable dans le temps. L'influence de l'indice de réfraction propre à l'agent chimique sur l'image capturée est également limité par l'utilisation de l'ombroscopie, plus particulièrement à l'aide d'un objectif télécentrique préférentiellement couplé à un illuminateur collimaté. Un autre intérêt de l'ombroscopie utilisé avec des milieux d'identification tel que des milieux chromogènes est également de pouvoir réaliser de façon simultanée une étape d'identification des particules biologiques, typiquement du type de microorganismes en présence et d'une étape d'estimation d'une valeur de concentration minimale inhibitrice et ce à partir d'un seul milieu de culture et d'une unique étape de captation d'image.

Les figures 35a, 35b, 35c, sont des images captées par différents dispositifs de détection. La figure 35a est obtenue par un système décrit dans la demande de brevet internationale WO 2012/152768 (référence *SIU*). L'éclairage utilisé pour la captation d'image est un éclairage annulaire bas, constitué de quatre barres de dix LED RGB disposées en aplomb du milieu de culture et orientées en direction de la surface du milieu de culture avec un angle de 45°. Les quatre barres sont réparties régulièrement à 90° autour du milieu. Le capteur utilisé pour la captation d'image est un capteur CMOS de 5 mégapixels, chaque pixels représentant une surface de $3.45\mu m$ par $3.45\mu m$. Un fond noir est disposé sous le milieu de culture pour la captation d'images. Seul le canal R (rouge) des images captées est représenté. La figure 35b est obtenue à l'aide d'un appareil « Bio-System Incubator » commercialisé par la société ADVENCIS et décrit dans la demande de brevet internationale WO 2013/110734 (référence *Advencis*), constitué d'un scanner présentant un éclairage linéaire en réflexion et d'une caméra linéaire. La figure 35c est obtenue à l'aide du dispositif illustré sur la figure 9 (référence *OMBRO*). Pour chacune de ces images, un milieu de culture Mueller Hinton E (bioMérieux MHE) est ensemencé par inondation avec une suspension calibrée à 0,5 Mc Farland d'*Escherichia Coli.* Une bandelette Etest® (bioMérieux) présentant un gradient de gentamicine est déposée à la surface de chaque milieu. Chacune des images est obtenue à un temps de mesure et d'incubation à 37°C $T_1$ de six heures. Afin de quantifier la différence de contraste obtenue, une zone A, visible sur la figure 35c, dont la largeur est délimitée par les graduations « 192 » et « 256 » de chaque bandelette selon un axe Y et longue de 50 mm selon l'axe X est définie. Cette zone est perpendiculaire à la bandelette qui s'étend alors parallèlement à l'axe Y. Un profil selon X est alors obtenu en effectuant pour chaque position en X la moyenne des valeurs de gris des pixels selon Y dans la zone A. Les profils ainsi obtenus sont présentés sur la figure 36 pour chacun des dispositifs de captation d'image avec les références respectives, SIU, ADVENCIS et OMBRO.

La figure 36 est ainsi une illustration schématique de trois profils de niveaux de gris (NG, codés sur 8bits) observés sur des images captées par les trois dispositifs de détection, , « SIU » pour la figure 35a, « Advencis » pour la figure 35b et « OMBRO » pour la figure 35c. Il est visible sur chaque profil de la figure 36 la transition entre le tapis bactérien, la zone de croissance, et la zone inhibition, ainsi qu'entre la zone d'inhibition et la bandelette Etest®. Afin d'estimer le contraste obtenu par chaque dispositif, on définit Imax comme la valeur de gris maximale observée dans la zone du tapis bactérien et Imin comme la valeur de gris minimale observée dans la zone d'inhibition. Ainsi on calcule un contraste selon l'équation :

$$\text{Contraste} = (Imax - Imin) / (Imax + Imin)$$

Le tableau 1 suivant donne les résultats de contraste pour les trois profils :

Tableau 1

|          | Ombro  | SIU   | Advencis |
|----------|--------|-------|----------|
| Imin     | 85     | 118   | 47       |
| Imax     | 190    | 139   | 54       |
| **Contraste** | **38,18%** | **8,17%** | **6,93%** |

Les résultats de contraste pour les trois profils démontrent que le dispositif OMBRO par ombroscopie, plus particulièrement à l'aide d'un objectif télécentrique préférentiellement couplé à un illuminateur collimaté, permet d'obtenir une valeur de contraste de 38,18%, bien supérieure aux autres techniques et ce dès six heures d'incubation. De plus la valeur de concentration minimale inhibitrice observée et bien conforme aux recommandations des organismes de régulations pour ce couple antibiotique / microorganisme.

Les figures 37a, 37b, 37c, sont des images captées par un dispositif de détection, « SIU », d'une suspension d'*Escherichia coli* (ATCC « American Type Culture Collection » 25922) sur un milieu de culture Mueller Hinton E (bio-Mérieux MHE), en présence de Gentamicine après incubation à un temps de mesure $T_1$ respectivement de quatre heures, six heures et vingt-quatre heures. Seul le canal R (rouge) des images captées est représenté.

Les figures 38a, 38b, 38c, sont des images captées par un dispositif de détection, par ombroscopie selon la figure 9, d'une suspension d'*Escherichia coli* (ATCC 25922) sur un milieu de culture Mueller Hinton E (bioMérieux MHE), en présence de Gentamicine après incubation à un temps de mesure $T_1$ respectivement de trois heures trente minutes, six heures et vingt-quatre heures.

Les figure 39a et 39b sont des illustrations schématiques de trois profils de niveaux de gris (NG, codés sur 8bits) observés sur des images captées à chaque temps de mesure pour chacun des dispositifs utilisé pour l'obtention des figures 37a, 37b et 37c et 38a, 38b, 38c.

Le tableau 2 suivant donne les résultats de contraste pour les trois profils à chaque temps d'incubation :

Tableau 2

| 3H30      | Ombro  | SIU   | 4H   |
|-----------|--------|-------|------|
| Imin      | 70     | 87    |      |
| Imax      | 165    | 87    |      |
| **Contraste** | 40,43% | 0,00% |      |
| **6H**    | **Ombro** | **SIU** | 6H |
| Imin      | 70     | 87    |      |
| Imax      | 180    | 95    |      |
| **Contraste** | 44,00% | 4,40% |      |
| **24H**   | **Ombro** | **SIU** | 24H |
| Imin      | 70     | 87    |      |
| Imax      | 190    | 110   |      |
| **Contraste** | 46,15% | 11,68% |      |

On montre ainsi que l'ombroscopie génère d'avantage de contraste que le dispositif d'imagerie SIU. En effet dès trois heures trente minutes le niveau de contraste observé est de plus de 40% alors que même après quatre heures d'incubation le dispositif SIU ne présente aucun contraste et donc aucune zone d'inhibition n'est déterminable de manière fiable et répétable. De plus, même après vingt-quatre heures d'incubation le niveau de contraste n'est que de 11,68% ce qui peut entrainer une fausse détermination d'une concentration minimale inhibitrice et est inacceptable dans le processus de décision clinique mettant en jeu la santé de patients. Les profils de niveau de gris obtenus par ombroscopie, particulièrement à l'aide d'un objectif télécentrique préférentiellement couplé à un illuminateur collimaté présentent donc des contrastes bien plus importants, ce qui permet également une analyse par des moyens de calcul, notamment une étape de mesure 104 et de traitement 105 automatisée plus fiable et répétable.

Les procédés et dispositifs décrits dans la présente invention peuvent être mis en oeuvre par un ou plusieurs programmes d'ordinateur, qui peuvent être présentés sous des formes diverses, actifs ou inactifs, sur une unique ordinateur ou répartis sur des systèmes d'ordinateur. Par exemple, ils peuvent être mis en oeuvre par des logiciels comprenant des instructions aptes à mettre en oeuvre les procédés de la présente invention et décrits sous forme de code source, code objet, code exécutable ou tout format permettant la réalisation de certaines étapes des procédés selon l'invention, notamment les étapes 104 de mesure et/ou 105 de traitement. Tous ces programmes d'ordinateur peuvent être stockés sur un support lisible de stockage pour ordinateur, ce qui inclus les supports de stockages et signaux correspondant, sous une forme compressée ou décompressée.

Le terme ordinateur se réfère à tout dispositif électronique comprenant un processeur, tel qu'une unité centrale de traitement (CPU), un processeur dédié ou un microcontrôleur. Un ordinateur est capable de recevoir des données (une ou des entrées), de réaliser une séquence d'étapes prédéterminées sur ces données, et de produire un résultat sous la forme d'information ou de signaux (une ou des sorties). Selon le contexte, le terme ordinateur peut signifier un processeur en particulier ou plus généralement un processeur associé à un assemblage d'éléments interconnectés contenus dans un boitier unique.

Le terme support lisible de stockage pour ordinateur ou support de stockage se réfère à tout moyen de contenir, stocker, communiquer, distribuer, ou transporter le programme d'ordinateur pour son utilisation par ou en relation avec un ordinateur ou tout moyen d'exécution dudit programme. Le support lisible de stockage pour ordinateur peut être, de manière non limitative, un système électronique, magnétique, optique, électromagnétique, infrarouge ou à semi-conducteur ainsi qu'un appareil, dispositif ou moyen de propagation dudit programme. Des exemples plus spécifiques et non limitatifs de supports de stockage peuvent être une disquette, un cédérom, de la mémoire vive (RAM), de la mémoire morte (ROM), de la mémoire morte intégrée programmable (EPROM ou stockage FLASH), une fibre optique ou encore toute connexion électrique comprenant un ou plusieurs câbles.

**Revendications**

1. Procédé de détection (100) d'une présence ou d'une absence d'au moins une première zone d'inhibition ($Z_1$) que comprend une culture de particules biologiques (1) sur un milieu de culture (2) en présence d'un agent chimique (3), le procédé de détection comprenant une première étape d'ensemencement (101) du milieu de culture (2) avec les particules biologiques (1), une deuxième étape de réception (102) par le milieu de culture (2) d'un support imprégné (5,5',5",5"',5"") de l'agent chimique (3), une troisième étape d'incubation (103) du milieu de culture, **caractérisé en ce que** le procédé de détection (100) comprend une quatrième étape de mesure (104) comportant

   - une première phase ($X_1$) de prise d'une image du milieu de culture (2) par ombroscopie de sorte à obtenir une image bidimentionnelle d'intensité (7) dudit milieu dans laquelle la première zone d'inhibition (Z1) est plus claire qu'une zone de prolifération (Z2) des particules biologiques (1) ; et
   - une analyse de l'image bidimentionnelle d'intensité (7) de sorte à détecter la présence ou l'absence de la première zone d'inhibition ($Z_1$)

2. Procédé de détection (100) selon la revendication précédente, **caractérisé en ce que** la quatrième étape de mesure (104) comprend une mesure d'une distance (F) entre le support imprégné (5,5',5",5"',5"") et un bord (B) de la première zone d'inhibition ($Z_1$).

3. Procédé de détection (100) selon la revendication 1, **caractérisé en ce que** la quatrième étape de mesure (104) comprend une mesure d'un diamètre (D) de la première zone d'inhibition ($Z_1$).

4. Procédé de détection (100) selon l'une quelconque des revendications 2 et 3, **caractérisé en ce que** le procédé de détection (100) comprend une cinquième étape de traitement (105) qui comporte une deuxième phase ($X_2$) de prise d'un cliché témoin (7') du milieu de culture (2).

5. Procédé de détection (100) selon la revendication 4, **caractérisé en ce que** le cliché témoin (7') est le cliché d'une règle (15) disposée sur le milieu de culture (2).

6. Procédé de détection (100) selon l'une quelconque des revendications 4 et 5, **caractérisé en ce que** le procédé de détection (100) comprend une troisième phase ($X_3$) de superposition de l'image (7) et du cliché témoin (7') pour former une représentation normée (7").

**7.** Procédé de détection (100) selon l'une quelconque des revendications 4 à 6, **caractérisé en ce que** la cinquième étape de traitement (105) comprend une quatrième phase ($X_4$) de localisation d'un repère (16) de la règle (15).

**8.** Procédé de détection (100) selon l'une quelconque des revendications 4 à 7, **caractérisé en ce que** la cinquième étape de traitement (105) comprend une cinquième phase ($X_5$) de traitement de la représentation normée (7").

**9.** Procédé de détection (100) selon la revendication 8, **caractérisé en ce que** la cinquième phase ($X_5$) comprend au moins une opération de lissage pour homogénéiser la représentation normée (7").

**10.** Procédé de détection (100) selon l'une quelconque des revendications 8 et 9, **caractérisé en ce que** la cinquième phase ($X_5$) comprend au moins une opération de dilatation dynamique de la représentation normée (7") qui comporte un étalage de la représentation normée (7") pour former un histogramme (17) d'un contraste de la représentation normée (7").

**11.** Procédé de détection (100) selon l'une quelconque des revendications 8 à 10, **caractérisé en ce que** la cinquième phase ($X_5$) comprend au moins une opération de seuillage de la représentation normée (7") qui comporte une détection d'un seuil (19) et une détermination d'un contour (20) de la représentation normée (7").

**12.** Procédé de détection (100) selon l'une quelconque des revendications 3 ou 8 à 11, **caractérisé en ce qu'**il comprend au moins une opération d'estimation d'une concentration minimale d'inhibition (CMI), pour déterminer une susceptibilité des particules biologiques (1) envers l'agent chimique (3).

**13.** Procédé de détection (100) selon l'une quelconque des revendications 4 à 12, **caractérisé en ce que** la première phase ($X_1$) de prise de l'image (7) et la deuxième phase ($X_2$) de prise du cliché témoin (7') sont des phases réalisées en continu.

**14.** Procédé de détection (100) selon l'une quelconque des revendications 4 à 12, **caractérisé en ce que** la première phase ($X_1$) de prise de l'image (7) et la deuxième phase ($X_2$) de prise du cliché témoin (7') sont des phases réalisées à un laps de temps déterminé.

**15.** Dispositif de détection (12) pour la mise en œuvre d'un procédé selon l'une quelconque des revendications précédentes, **caractérisé en ce qu'**il comprend :

- une source lumineuse (8) configurée pour produire des rayons lumineux (9) collimatés en direction d'un milieu de culture de particules biologiques (2) ;
- un foyer lumineux (8') configuré pour éclairer directement ledit milieu de culture (2) ;
- un moyen de captation (6) configuré pour prendre par ombroscopie une image bidimensionnelle d'intensité (7) dudit milieu de culture (2) dans laquelle la première zone d'inhibition (Z1) est plus claire qu'une zone de prolifération (Z2) des particules biologiques (1); et
- des moyens de calcul (14) configuré pour analyser ladite image (7) de sorte à détecter la présence ou l'absence d'au moins une première zone d'inhibition des particules biologiques (Z1) sur ledit milieu de culture (2).

**16.** Dispositif de détection (12) selon la revendication 15, **caractérisé en ce que** le moyen de captation (6) comporte un axe de captation (A) qui est ménagé orthogonalement par rapport à un premier plan ($P_1$) selon lequel est étendu le milieu de culture (2).

**17.** Dispositif de détection (12) selon la revendication 15, **caractérisé en ce que** la source lumineuse (8) est placée sur l'axe de captation (A), le milieu de culture (2) étant interposé entre le moyen de captation (6) et la source lumineuse (8).

**18.** Dispositif de détection (12) selon la revendication 15, **caractérisé en ce que** le dispositif de détection (12) comprend au moins un miroir semi-réfléchissant (10) pour renvoyer les rayons lumineux (9) vers le milieu de culture (2).

**19.** Dispositif de détection (12) selon l'une des revendications 15 à 18, **caractérisé en ce que** le moyen de captation (6) comprend un objectif télécentrique (11).

**Patentansprüche**

1. Verfahren (100) zur Erfassung des Vorhandenseins oder Nichtvorhandenseins mindestens eines ersten Sperrbereichs ($Z_1$), den eine Kultur biologischer Partikel (1) auf einem Kulturmedium (2) in Gegenwart eines chemischen Arbeitsstoffs (3) enthält, wobei das Erfassungsverfahren einen ersten Schritt de,s Impfens (101) des Kulturmediums (2) mit den biologischen Partikeln (1), einen zweiten Schritt des Empfangs (102) durch das Kulturmedium (2) einer mit dem chemischen Arbeitsstoff (3) durchtränkten Trägersubstanz (5, 5', 5", 5"', 5"''), einen dritten Schritt der Inkubation (103) des Kulturmediums enthält, **dadurch gekennzeichnet, dass** das Erfassungsverfahren (100) einen vierten Messschritt (104) enthält, der aufweist

   - eine erste Phase ($X_1$) der Aufnahme eines Bilds des Kulturmediums (2) durch Schattenaufnahme, um ein zweidimensionales Intensitätsbild (7) des Mediums zu erhalten, in dem der erste Sperrbereich (Z1) heller ist als ein Proliferationsbereich (Z2) der biologischen Partikel (1); und
   - eine Analyse des zweidimensionalen Intensitätsbilds (7), um das Vorhandensein oder das Nichtvorhandensein des ersten Sperrbereichs ($Z_1$) zu erfassen.

2. Erfassungsverfahren (100) nach dem vorhergehenden Anspruch, **dadurch gekennzeichnet, dass** der vierte Messschritt (104) eine Messung eines Abstands (F) zwischen der durchtränkten Trägersubstanz (5, 5', 5", 5"', 5"'') und einem Rand (B) des ersten Sperrbereichs (Z1) enthält.

3. Erfassungsverfahren (100) nach Anspruch 1, **dadurch gekennzeichnet, dass** der vierte Messschritt (104) eine Messung eines Durchmessers (D) des ersten Sperrbereichs ($Z_1$) enthält.

4. Erfassungsverfahren (100) nach einem der Ansprüche 2 und 3, **dadurch gekennzeichnet, dass** das Erfassungsverfahren (100) einen fünften Verarbeitungsschritt (105) enthält, der eine zweite Phase ($X_2$) der Aufnahme eines Kontrollfotos (7') des Kulturmediums (2) enthält.

5. Erfassungsverfahren (100) nach Anspruch 4, **dadurch gekennzeichnet, dass** das Kontrollfoto (7') das Foto eines auf dem Kulturmedium (2) angeordneten Lineals (15) ist.

6. Erfassungsverfahren (100) nach einem der Ansprüche 4 und 5, **dadurch gekennzeichnet, dass** das Erfassungsverfahren (100) eine dritte Phase ($X_3$) des Übereinanderlegens des Bilds (7) und des Kontrollfotos (7') enthält, um eine normierte Darstellung (7") zu formen.

7. Erfassungsverfahren (100) nach einem der Ansprüche 4 bis 6, **dadurch gekennzeichnet, dass** der fünfte Verarbeitungsschritt (105) eine vierte Phase ($X_4$) der Lokalisierung eines Festpunkts (16) des Lineals (15) enthält.

8. Erfassungsverfahren (100) nach einem der Ansprüche 4 bis 7, **dadurch gekennzeichnet, dass** der fünfte Verarbeitungsschritt (105) eine fünfte Phase ($X_5$) der Verarbeitung der normierten Darstellung (7") enthält.

9. Erfassungsverfahren (100) nach Anspruch 8, **dadurch gekennzeichnet, dass** die fünfte Phase ($X_5$) mindestens einen Glättungsvorgang enthält, um die normierte Darstellung (7") zu homogenisieren.

10. Erfassungsverfahren (100) nach einem der Ansprüche 8 und 9, **dadurch gekennzeichnet, dass** die fünfte Phase ($X_5$) mindestens einen Vorgang der dynamischen Ausdehnung der normierten Darstellung (7") enthält, der eine Ausbreitung der normierten Darstellung (7") aufweist, um ein Histogramm (17) eines Kontrasts der normierten Darstellung (7") zu formen.

11. Erfassungsverfahren (100) nach einem der Ansprüche 8 bis 10, **dadurch gekennzeichnet, dass** die fünfte Phase ($X_5$) mindestens einen Vorgang der Schwellwertbildung der normierten Darstellung (7") enthält, der eine Erfassung eines Schwellwerts (19) und eine Bestimmung eines Umrisses (20) der normierten Darstellung (7") aufweist.

12. Erfassungsverfahren (100) nach einem der Ansprüche 3 oder 8 bis 11, **dadurch gekennzeichnet, dass** es mindestens einen Vorgang der Schätzung einer minimalen Sperrkonzentration (CMI) enthält, um eine Empfindlichkeit der biologischen Partikel (1) gegenüber dem chemischen Arbeitsstoff (3) zu bestimmen.

13. Erfassungsverfahren (100) nach einem der Ansprüche 4 bis 12, **dadurch gekennzeichnet, dass** die erste Phase ($X_1$) der Aufnahme des Bilds (7) und die zweite Phase ($X_2$) der Aufnahme des Kontrollfotos (7') fortlaufend durchgeführte Phasen sind.

**14.** Erfassungsverfahren (100) nach einem der Ansprüche 4 bis 12, **dadurch gekennzeichnet, dass** die erste Phase ($X_1$) der Aufnahme des Bilds (7) und die zweite Phase ($X_2$) der Aufnahme des Kontrollfotos (7') Phasen sind, die in einem bestimmten Zeitraum durchgeführt werden.

**15.** Erfassungsvorrichtung (12) zur Durchführung eines Verfahrens nach einem der vorhergehenden Ansprüche, **dadurch gekennzeichnet, dass** sie enthält:

- eine Lichtquelle (8), die konfiguriert ist, kollimierte Lichtstrahlen (9) in Richtung eines Kulturmediums von biologischen Partikeln (2) zu erzeugen;
- einen Lichtherd (8'), der konfiguriert ist, das Kulturmedium (2) direkt zu beleuchten;
- eine Auffangeinrichtung (6), die konfiguriert ist, durch Schattenaufnahme ein zweidimensionales Intensitätsbild (7) des Kulturmediums (2) aufzunehmen, bei dem der erste Sperrbereich (Z1) heller ist als ein Proliferationsbereich (Z2) der biologischen Partikel (1); und
- Recheneinrichtungen (14), die konfiguriert sind, das Bild (7) zu analysieren, um das Vorhandensein oder Nichtvorhandensein mindestens eines ersten Sperrbereichs der biologischen Partikel (Z1) auf dem Kulturmedium (2) zu erfassen.

**16.** Erfassungsvorrichtung (12) nach Anspruch 15, **dadurch gekennzeichnet, dass** die Auffangeinrichtung (6) eine Auffangachse (A) aufweist, die orthogonal bezüglich einer ersten Ebene ($P_1$) eingerichtet ist, gemäß der sich das Kulturmedium (2) erstreckt.

**17.** Erfassungsvorrichtung (12) nach Anspruch 15, **dadurch gekennzeichnet, dass** die Lichtquelle (8) auf der Auffangachse (A) angeordnet ist, wobei das Kulturmedium (2) zwischen die Auffangeinrichtung (6) und die Lichtquelle (8) eingefügt ist.

**18.** Erfassungsvorrichtung (12) nach Anspruch 15, **dadurch gekennzeichnet, dass** die Erfassungsvorrichtung (12) mindestens einen halbreflektierenden Spiegel (10) aufweist, um die Lichtstrahlen (9) zum Kulturmedium (2) zurückzuschicken.

**19.** Erfassungsvorrichtung (12) nach einem der Ansprüche 15 bis 18, **dadurch gekennzeichnet, dass** die Auffangeinrichtung (6) ein telezentrisches Objektiv (11) enthält.

## Claims

**1.** A method (100) for detecting the presence or absence of at least one first inhibition zone ($Z_1$) comprised by a culture of biological particles (1) on a culture medium (2) in the presence of a chemical agent (3), said method of detection comprising a first step (101) of seeding the culture medium (2) with the biological particles (1), a second step (102) in which the culture medium (2) receives a support (5,5',5",5"',5"") impregnated with the chemical agent (3), and a third step of incubation (103) of the culture medium, **characterized in that** the method of detection (100) comprises a fourth step of measurement (104) comprising:

- a first phase ($X_1$) of taking an image (7) of the culture medium (2) by ombroscopy so as to generate a two-dimensional intensity image (7) of the culture medium in which the first inhibition zone (Z1) is brighter than a proliferation zone (Z2) of the biological particles (1);
- an analysis of the two-dimensional intensity image (7) so as to detect the presence or the absence of the first inhibition zone (Z1).

**2.** The method of detection (100) as claimed in the preceding claim, **characterized in that** the fourth step of measurement (104) comprises measurement of a distance (F) between the impregnated support (5,5',5",5"',5"") and an edge (B) of the first inhibition zone ($Z_1$).

**3.** The method of detection (100) as claimed in claim 1, **characterized in that** the fourth step of measurement (104) comprises measurement of a diameter (D) of the first inhibition zone ($Z_1$).

**4.** The method of detection (100) as claimed in either of claims 2 and 3, **characterized in that** the method of detection (100) comprises a fifth processing step (105), which comprises a second phase ($X_2$) of taking a control photograph (7') of the culture medium (2).

5. The method of detection (100) as claimed in claim 4, **characterized in that** the control photograph (7') is a photograph of a ruler (15) arranged on the culture medium (2).

6. The method of detection (100) as claimed in either of claims 4 and 5, **characterized in that** the method of detection (100) comprises a third phase ($X_3$) of superimposing the image (7) and the control photograph (7') to form a normed representation (7").

7. The method of detection (100) as claimed in any one of claims 4 to 6, **characterized in that** the fifth processing step (105) comprises a fourth phase ($X_4$) of localization of a reference (16) of the ruler (15).

8. The method of detection (100) as claimed in any one of claims 4 to 7, **characterized in that** the fifth processing step (105) comprises a fifth phase ($X_5$) of processing the normed representation (7").

9. The method of detection (100) as claimed in claim 8, **characterized in that** the fifth phase ($X_5$) comprises at least one smoothing operation for homogenizing the normed representation (7").

10. The method of detection (100) as claimed in either of claims 8 and 9, **characterized in that** the fifth phase ($X_5$) comprises at least one operation of dynamic stretching of the normed representation (7"), which comprises spreading of the normed representation (7") to form a histogram (17) of a contrast of the normed representation (7").

11. The method of detection (100) as claimed in any one of claims 8 to 10, **characterized in that** the fifth phase ($X_5$) comprises at least one thresholding operation of the normed representation (7"), which comprises detection of a threshold (19) and determination of a contour (20) of the normed representation (7").

12. The method of detection (100) as claimed in any one of claims 3 or 8 to 11, **characterized in that** it comprises at least one operation of estimating a minimum inhibitory concentration (MIC), to determine sensitivity of the biological particles (1) to the chemical agent (3).

13. The method of detection (100) as claimed in any one of claims 4 to 12, **characterized in that** the first phase ($X_1$) of taking the image (7) and the second phase ($X_2$) of taking the control photograph (7') are phases that are performed continuously.

14. The method of detection (100) as claimed in any one of claims 4 to 12, **characterized in that** the first phase ($X_1$) of taking the image (7) and the second phase ($X_2$) of taking the control photograph (7') are phases that are performed with a defined time lapse.

15. A device (12) for carrying out a method as claimed in any one of the preceding claims, **characterized in that** it comprises:

   - a light source (8) configured to produce collimated light rays(9) toward a biological particle culture medium (2);
   - an optical focus (8') configured to directly illuminate said culture medium (2);
   - sensing means (6) configured to acquire a two-dimensional intensity image (7) of said culture medium (2) in which the first inhibition zone (Z1) is brighter than a proliferation zone (Z2) of the biological particles (1); and
   - computation means (14) configured to analyse said image (7) so as to detect the presence or the absence of at least one first inhibition zone (Z1) of the biological particles (1) on said culture medium (2).

16. The detecting device (12) as claimed in claim 15, **characterized in that** the sensing means (6) comprises a sensing axis (A) that is arranged orthogonally relative to a first plane ($P_1$) over which the culture medium (2) extends.

17. The detecting device (12) as claimed in claim 15, **characterized in that** the light source (8) is positioned on the sensing axis (A), the culture medium (2) being interposed between the sensing means (6) and the light source (8).

18. The detecting device (12) as claimed in claim 15, **characterized in that** the detecting device (12) comprises at least one semireflecting mirror (10) for returning the light rays (9) to the culture medium (2).

**19.** The detecting device (12) as claimed in any one of claims 15 to 18, **characterized in that** the sensing means (6) comprises a telecentric objective (11).

Fig.1

Fig.3

Fig.2

Fig.4

T0

Fig.5

T1

Fig.6

T2

Fig.4bis

T0

Fig.5bis

T1

Fig.6bis

T2

Fig.4ter

Fig.5ter

Fig.6ter

Fig.7

Fig.8

Fig.9

Fig.10

Fig.11a

Fig.11b

Fig.11c

Fig.12a

Fig.12b

Fig.12c

Fig.13a

Fig.13b

Fig.14a    Fig.14b    Fig.14c    Fig.14d    Fig.14e    Fig.14f

Fig.15a    Fig.15b    Fig.15c    Fig.15d    Fig.15e    Fig.15f

Fig.16a    Fig.16b    Fig.16c    Fig.16d    Fig.16e    Fig.16f

Fig.17

Fig.18

Fig.19

Fig.20

Fig.21

Fig.22

Fig.23

Fig.24

$$CMI = \frac{256}{2^{\frac{|X_{CMI}-X_{256}|}{div}}}$$

Fig.25

Fig.26

Fig.27

Fig.28

Fig.29

Fig.30

Fig. 31a                    Fig. 31b                    Fig. 31c

Fig. 32a                    Fig. 32b                    Fig. 32c

Fig. 33a

Fig. 33b

Fig. 33c

Fig. 34a

Fig. 34b

Fig. 34c

Fig. 35a

Fig. 35b

Fig. 35c

Fig. 36

Fig. 37a

Fig 37b

Fig 37c

Fig. 38a

Fig 38b

Fig 38c

Fig. 39a

Fig 39b

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011125033 A
- US 2003186350 A1
- EP 1016707 A2
- DE 202004000946 U1
- WO 2012152768 A
- WO 2013110734 A